# EUROPEAN PATENT SPECIFICATION

(11) **EP 3 662 808 B1**
(45) Date of publication and mention of the grant of the patent: **27.09.2023**
(21) Application number: 17919685.2
(22) Date of filing: 02.08.2017
(51) Int. Cl.: A61B 1/00, A61B 1/12, G02B 23/16, G02B 23/24

(54) **RIGID ENDOSCOPE COVER AND ENDOSCOPE UNIT**
STARRER ENDOSKOPSCHUTZ UND ENDOSKOPEINHEIT
GAINE D'ENDOSCOPE RIGIDE ET ENDOSCOPE

(43) Date of publication of application: 10.06.2020
(73) Proprietor: Nagasaki University, Nagasaki-Shi, Nagasaki 852-8521 (JP); Trytec Co., Ltd., Oita-shi, Oita 870-0278 (JP)
(72) Inventor: MATSUMOTO Keitaro, Nagasaki-shi Nagasaki 852-8521 (JP); NAGAYASU Takeshi, Nagasaki-shi Nagasaki 852-8521 (JP); TAKAGI Katsunori, Nagasaki-shi Nagasaki 852-8521 (JP); TAKEZAKI Hiroshi, Oita-shi Oita 870-0278 (JP); MORI Junji, Oita-shi Oita 870-0278 (JP)
(74) Representative: Ter Meer Steinmeister & Partner
(86) International application number: PCT/JP2017/028076
(87) International publication number: WO 2019/026206

(56) References cited:
- JP-A- 2012 187 138
- JP-A- 2015 177 914
- JP-U- 3 186 191
- US-A- 3 835 842
- US-A- 4 920 961
- US-A1- 2003 130 565
- US-A1- 2006 041 186

## Description

### TECHNICAL FIELD

The present invention relates to a hollow cover for covering the outer circumferential surface of a rigid endoscope having an observation window portion provided at a tip thereof to protect an objective lens for taking an image of an affected area, and an endoscope unit including the rigid endoscope and the cover.

### BACKGROUND ART

In recent years, in the medical field, endoscopes (soft endoscopes) represented by a stomach camera have been used in examination and surgery of the stomach. In many cases, a thoracoscope is used in surgery performed with the chest opened, and a laparoscope is used in surgery performed with the abdomen opened (hereinafter, referred to as laparoscopic surgery). Laparoscopic surgery is rapidly spreading due to its low invasiveness and good aesthetic outcome, and is mainly used for gastric cancer and colon cancer surgery, and the proportion of laparoscopic surgery to total surgery is increasing year by year. Meanwhile, the above-described thoracoscope and laparoscope, etc., are called rigid endoscopes. At a tip of a rigid endoscope, a soft endoscope, or the like, an objective lens for taking an image of an affected area is provided, and an observation window portion is also provided to protect the objective lens.

During surgery using a rigid endoscope such as the above-described thoracoscope or laparoscope, the observation window portion provided at the tip of the rigid endoscope often becomes cloudy or becomes dirty with blood, fat, etc. For this reason, there is a problem that the surgery has to be temporarily interrupted for washing or cleansing the observation window portion and thus the surgery efficiency is significantly reduced. To explain in more detail, information required for the surgery using the rigid endoscope is only image (visual) information obtained by the rigid endoscope, and cloudiness of the observation window portion or dirt on the observation window portion due to blood, fat, etc., during the surgery hinders the surgery from being safely performed. For this reason, it is necessary to take out the rigid endoscope from the body and wash the rigid endoscope during the surgery, but the time required for this leads to an increase in the surgery time, which may cause a heavy burden on the patient or an unexpected complication.

In order to solve such a problem, Patent Literature 1 discloses a technology of mounting a washing device (specifically, a long tube), which supplies washing water such as physiological saline and gas for blowing away the washing water to the observation window portion of a rigid endoscope within the body of a patient, on the outer circumferential surface of the rigid endoscope. In addition, Patent Literatures 2 to 5 each disclose a technology of overlaying a washing sheath, within which a supply passage for washing water and gas is provided, on a rigid endoscope, thereby allowing an observation window portion to be washed within the body of a patient.

### CITATION LIST

### [PATENT LITERATURE]

[PTL 1] Japanese Utility Model Registration No. 3186191
[PTL 2] Japanese Laid-Open Patent Publication No. 2012-254188
[PTL 3] Japanese Laid-Open Patent Publication No. 2015-177914
[PTL 4] Japanese Patent No. 2539980
[PTL 5] Japanese Laid-Open Patent Publication No. H5-207962

JP 2012187138A discloses a rigid endoscope that can include a conduit that flows a fluid to wash an observation window disposed at the distal end of the insertion part of the rigid endoscope without enlarging the diameter of the insertion part, and in which the washing of the conduit is unnecessarily or can be easily performed.

US 4,920,961A discloses a sheath having a tube and a latch receptacle for removably connecting the sheath with a cooperating working element. The latch receptacle is static and has means for receiving at least a portion of a movable latch of the working element such that the latch receptacle can be disconnected from the working element without touching the sheath. A continuous flow resectoscope sheath assembly may also be provided with both an inlet post and an outlet post on the outer sheath portion.

### SUMMARY OF THE INVENTION

However, in the case where a long tube is mounted on the outer circumferential surface of a rigid endoscope as a washing device which supplies washing water and gas to the observation window portion of the rigid endoscope as disclosed in Patent Literature 1, when the rigid endoscope is inserted into the body of a patient, the long tube may be detached from the rigid endoscope within the body of the patient, and the inside of the body of the patient may be damaged by the detached long tube. In addition, there is a problem that it is necessary to wash the long tube every time before performing surgery using the rigid endoscope, which takes time and effort. Furthermore, with the washing device disclosed in Patent Literature 1, there is a problem that a step at a tube-mounted portion may hinder insertion into the body of a patient, or a tube bent portion at the tip has an acute angle and high rigidity and thus may damage an organ within the body of the patient.

Moreover, in the case where a washing sheath within which a supply passage for washing water and gas is provided is overlaid on the entire circumference in the circumferential direction of the outer circumferential surface of a rigid endoscope as disclosed in Patent Literatures 2 to 5, there is a problem that it takes time to overlay the washing sheath on the outer circumferential surface of the rigid endoscope. Furthermore, in the case where the supply passage for washing water and gas penetrates the inside of the washing sheath, there is a problem that the production cost of the washing sheath is increased.

The present invention has been made in view of such circumstances, and an object of the present invention is to provide: a rigid endoscope cover that is capable of washing an observation window portion within the body of a patient, that is capable of being easily mounted onto or detached from the outer circumferential surface of a rigid endoscope, and that can have reduced production cost; and an endoscope unit.

The invention is defined by claim 1. Preferred embodiments are defined by the dependent claims. Further embodiments disclosed herein are for exemplary purpose only and do not form part of the claimed invention.

### BRIEF DESCRIPTION OF THE DRAWINGS

FIG. 1 is a perspective view schematically illustrating the structure of a rigid endoscope in an endoscope unit;
FIG. 2 is a perspective view illustrating the structure of a rigid endoscope cover in the endoscope unit;
FIG. 3 is a perspective view illustrating a state where the cover shown in FIG. 2 is overlaid on the rigid endoscope shown in FIG. 1;
FIG. 4 is a cross-sectional view of the endoscope unit shown in FIG. 3, taken along a virtual plane A;
FIG. 5 is a cross-sectional view of the endoscope unit shown in FIG. 3, taken along a virtual plane passing through the center line of the rigid endoscope;
FIG. 6 is a cross-sectional view illustrating a state where a cover is overlaid on a rigid endoscope;
FIG. 7 is a perspective view illustrating the structure of a rigid endoscope cover according to a first embodiment of the present invention;
FIG. 8 is a perspective view illustrating a state where the cover shown in FIG. 7 is overlaid on the rigid endoscope shown in FIG. 1;
FIG. 9 is a perspective view illustrating the structure of a rigid endoscope cover according to a modification in the first embodiment of the present invention;
FIG. 10 is a perspective view illustrating the detailed structure of an inner cover portion in the cover shown in FIG. 9;
FIG. 11 is a perspective view illustrating a state where the cover shown in FIG. 9 is overlaid on the rigid endoscope shown in FIG. 1;
FIG. 12 is a perspective view illustrating the structure of an endoscope unit according to another modification in the first embodiment of the present invention;
FIG. 13 is a cross-sectional view of the endoscope unit shown in FIG. 12, taken along a virtual plane B;
FIG. 14 is a perspective view illustrating the detailed structure of an inner cover portion in a cover of the endoscope unit shown in FIG. 12;
FIG. 15 is a cross-sectional view of the endoscope unit shown in FIG. 12, taken along a virtual plane passing through the center line of the rigid endoscope;
FIG. 16 is a side view illustrating the structure of a cover of an endoscope unit according to still another modification in the first embodiment of the present invention;
FIG. 17 is a bottom view illustrating the structure of the cover shown in FIG. 16, as seen from below;
FIG. 18 is a cross-sectional view of the cover shown in FIG. 16, as seen from the direction of arrows P-P;
FIG. 19 is a perspective view schematically illustrating the structure of a rigid endoscope in an endoscope unit;
FIG. 20 is a perspective view illustrating the structure of a rigid endoscope cover;
FIG. 21 is a perspective view illustrating a state where the cover shown in FIG. 20 is overlaid on the rigid endoscope shown in FIG. 19;
FIG. 22 is a cross-sectional view of the endoscope unit shown in FIG. 21, taken along a virtual plane C;
FIG. 23 is a cross-sectional view of the endoscope unit shown in FIG. 21, taken along a virtual plane passing through the center line of the rigid endoscope;
FIG. 24 is a cross-sectional view illustrating a state where a cover is overlaid on a rigid endoscope;
FIG. 25 is a perspective view illustrating the structure of a rigid endoscope cover according to a second embodiment of the present invention;
FIG. 26 is a perspective view illustrating a state where the cover shown in FIG. 25 is overlaid on the rigid endoscope shown in FIG. 19;
FIG. 27 is a cross-sectional view of an endoscope unit in which the cover shown in FIG. 2 is overlaid on the rigid endoscope shown in FIG. 19;
FIG. 28 is a cross-sectional view of the endoscope unit shown in FIG. 27, taken along a virtual plane passing through the center line of the rigid endoscope;
FIG. 29 is a cross-sectional view of an endoscope unit according to still another example; and
FIG. 30 is a cross-sectional view of the endoscope unit shown in FIG. 29, taken along a virtual plane passing through the center line of the rigid endoscope.

### DESCRIPTION OF THE PREFERRED EMBODIMENTS

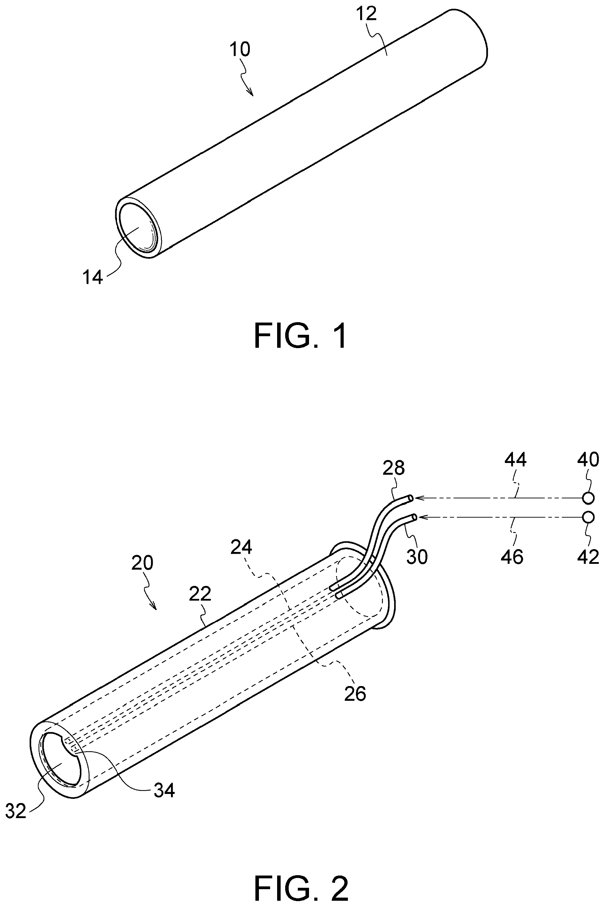

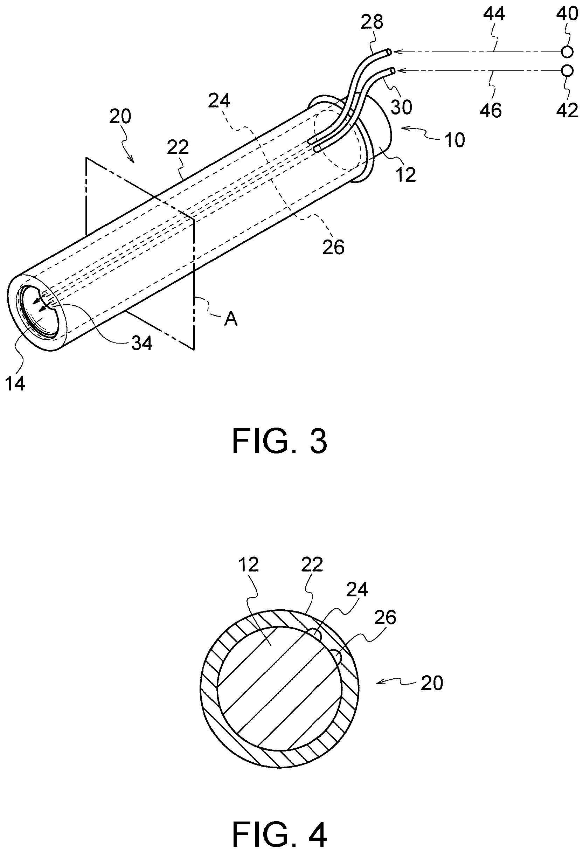

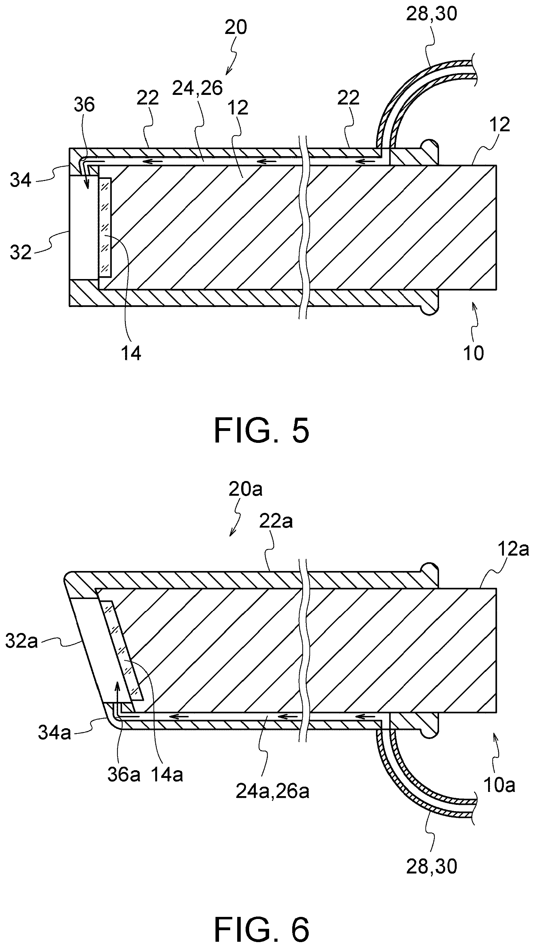

Hereinafter, a first embodiment of the present invention will be described with reference to the drawings. FIGS. 1 to 5 illustrate an endoscope unit according to the present embodiment. Among these figures, FIG. 1 is a perspective view schematically illustrating the structure of a rigid endoscope in the endoscope unit according to the present embodiment, and FIG. 2 is a perspective view illustrating the structure of a rigid endoscope cover in the endoscope unit. In addition, FIG. 3 is a perspective view illustrating a state where the cover shown in FIG. 2 is overlaid on the rigid endoscope shown in FIG. 1. Moreover, FIG. 4 is a cross-sectional view of the endoscope unit shown in FIG. 3, taken along a virtual plane A, and FIG. 5 is a cross-sectional view of the endoscope unit shown in FIG. 3, taken along a virtual plane passing through the center line of the rigid endoscope.

First, the rigid endoscope 10 will be described with reference to FIG. 1. As shown in FIG. 1, the rigid endoscope 10 has an elongated substantially cylindrical body portion 12, and an objective lens (not shown) for taking an image of an affected area and an observation window portion 14 such as a glass plate that protects the objective lens are provided at a tip of the body portion 12.

Next, a hollow cover 20 for covering the entirety in the circumferential direction of the outer circumferential surface of the rigid endoscope 10 will be described with reference to FIGS. 2 to 5. The material of the cover 20 is rubber such as natural rubber or synthetic rubber, and the cover 20 is overlaid on the outer circumferential surface of the rigid endoscope 10 shown in FIG. 1. More specifically, the cover 20 has an elongated substantially cylindrical body portion 22, and is overlaid on the outer circumferential surface of the rigid endoscope 10 by inserting a tip of the rigid endoscope 10 into a hollow portion at the base side (that is, the far side in FIG. 2) of the body portion 22. The endoscope unit is formed by combining the rigid endoscope 10 and the cover 20 described above. In addition, two grooves 24 and 26 are formed on the inner circumferential surface of the body portion 22 of the cover 20 so as to extend along the longitudinal direction of the body portion 22, and flow passages for washing fluids to be supplied to the observation window portion 14 of the rigid endoscope 10 are defined by the outer circumferential surface of the body portion 12 of the rigid endoscope 10 and the inner surfaces of the respective grooves 24 and 26. Here, of the two grooves 24 and 26, the groove 24 is used as a flow passage for washing water such as physiological saline to be supplied to the observation window portion 14 of the rigid endoscope 10, and the other groove 26 is used as a flow passage for gas for blowing away the washing water adhering to the observation window portion 14.

As shown in FIG. 2, an opening 32 is provided at a tip of the cover 20, and the observation window portion 14 is exposed to the outside of the cover 20 by the opening 32 when the cover 20 is overlaid on the outer circumferential surface of the rigid endoscope 10. In addition, a fluid direction change portion 34 that changes the direction of the washing fluids flowing along the respective grooves 24 and 26 (that is, along the longitudinal direction of the body portion 22 of the cover 20) to a direction toward the observation window portion 14 (that is, a direction toward the hollow portion of the cover 20), is provided near the opening 32 at the tip of the cover 20. Specifically, the fluid direction change portion 34 is provided with a curved portion 36 that defines the flow passages for the washing fluids, and the direction of the washing fluids is changed by the curved portion 36 from the left direction to a downward right direction in FIG. 5. Since the direction of the washing fluids flowing in the left direction in FIG. 5 along the outer circumferential surface of the body portion 12 of the rigid endoscope 10 can be changed by such a fluid direction change portion 34 to the direction toward the observation window portion 14 of the rigid endoscope 10, the observation window portion 14 can be reliably washed by the washing fluids. Moreover, such a fluid direction change portion 34 projects inward from the body portion 22 toward the hollow portion of the cover 20, and is formed such that the tip of the rigid endoscope 10 is caught on the fluid direction change portion 34 when the cover 20 is overlaid on the rigid endoscope 10. Therefore, the tip of the rigid endoscope 10 can be prevented from protruding from the tip of the cover 20.

As shown in FIGS. 3 and 5, fluid supply pipes 28 and 30 are respectively connected to the base end side (that is, the far side in FIG. 3) of the respective grooves 24 and 26 in the body portion 22 of the cover 20, and the washing water and the gas can be supplied from these fluid supply pipes 28 and 30 into the respective grooves 24 and 26. In addition, as shown in FIG. 2, a washing water supply source 40 is connected to the fluid supply pipe 28 via a washing water supply passage 44, and the washing water supplied from the washing water supply source 40 to the washing water supply passage 44 is sent to the fluid supply pipe 28. Therefore, when the cover 20 is overlaid on the rigid endoscope 10, the washing water can be supplied from the fluid supply pipe 28 to the flow passage defined between the outer circumferential surface of the body portion 12 of the rigid endoscope 10 and the inner surface of the groove 24 of the cover 20. Moreover, as shown in FIG. 2, a gas supply source 42 is connected to the fluid supply pipe 30 via a gas supply passage 46, and the gas supplied from the gas supply source 42 to the gas supply passage 46 is sent to the fluid supply pipe 30. Therefore, when the cover 20 is overlaid on the rigid endoscope 10, the gas can be supplied from the fluid supply pipe 30 to the flow passage defined between the outer circumferential surface of the body portion 12 of the rigid endoscope 10 and the inner surface of the groove 26 of the cover 20.

Next, a method for using the endoscope unit having such a structure will be described. First, in performing surgery in which the endoscope unit is inserted into the body of a patient, the cover 20 shown in FIG. 2 is overlaid on the outer circumferential surface of the rigid endoscope 10 shown in FIG. 1. Specifically, the tip of the rigid endoscope 10 is put into the hollow portion at the base side (that is, the far side in FIG. 2) of the body portion 22 of the cover 20, and the rigid endoscope 10 is inserted into the hollow portion of the cover 20 until the tip of the rigid endoscope 10 becomes caught on the fluid direction change portion 34 at the tip side of the cover 20. By doing so, the endoscope unit in which the cover 20 is overlaid on the rigid endoscope 10 as shown in FIG. 3 is formed. Then, the endoscope unit shown in FIG. 3 is inserted into the body of the patient, and the surgery is performed.

When the observation window portion 14 of the rigid endoscope 10 becomes dirty with blood, fat, etc., or becomes cloudy during the surgery, the observation window portion 14 is washed in a state where the endoscope unit has been inserted into the body of the patient. More specifically, by supplying the washing water from the washing water supply source 40 through the washing water supply passage 44 to the fluid supply pipe 28, the washing water flows in the left direction in FIG. 5 in the flow passage defined between the outer circumferential surface of the body portion 12 of the rigid endoscope 10 and the inner surface of the groove 24 of the cover 20. Then, the direction of the washing water flowing in the left direction in FIG. 5 as described above is changed at the tip of the cover 20 by the fluid direction change portion 34 to the direction toward the observation window portion 14. More specifically, the direction of the washing water flowing in the left direction in FIG. 5 along the flow passage defined between the outer circumferential surface of the body portion 12 of the rigid endoscope 10 and the inner surface of the groove 24 of the cover 20 is changed by the curved portion 36 of the fluid direction change portion 34 from the left direction to the downward right direction in FIG. 5. Therefore, the observation window portion 14 can be washed by the washing water. Thereafter, by supplying the gas from the gas supply source 42 through the gas supply passage 46 to the fluid supply pipe 30, the gas flows in the left direction in FIG. 5 in the flow passage defined between the outer circumferential surface of the body portion 12 of the rigid endoscope 10 and the inner surface of the groove 26 of the cover 20. Then, the direction of the gas flowing in the left direction in FIG. 5 as described above is changed at the tip of the cover 20 by the fluid direction change portion 34 to the direction toward the observation window portion 14. More specifically, the direction of the gas flowing in the left direction in FIG. 5 along the flow passage defined between the outer circumferential surface of the body portion 12 of the rigid endoscope 10 and the inner surface of the groove 26 of the cover 20 is changed by the curved portion 36 of the fluid direction change portion 34 from the left direction to the downward right direction in FIG. 5. Therefore, the washing water adhering to the observation window portion 14 can be blown away by the gas.

In the cover 20 having the structure as described above and the endoscope unit including such a cover 20 and the rigid endoscope 10, the grooves 24 and 26 are provided on the inner circumferential surface of the cover 20, and the flow passages for the washing fluids such as the washing water and the gas to be supplied to the observation window portion 14 of the rigid endoscope 10 are defined by the outer circumferential surface of the rigid endoscope 10 and the inner surfaces of the respective grooves 24 and 26. The observation window portion 14 is allowed to be washed within the body of a patient, by providing the grooves 24 and 26 as the flow passages for the washing fluids on the inner circumferential surface of the cover 20, which covers the outer circumferential surface of the rigid endoscope 10, as described above. Furthermore, since the grooves 24 and 26 are provided on the inner circumferential surface of the cover 20, the outer diameter of the rigid endoscope 10 covered with the cover 20 (that is, the endoscope unit) can be made relatively small, whereby the surgery efficiency can be improved and the inside of the body of a patient can be inhibited from being damaged.

Since such a cover 20 is merely overlaid on the rigid endoscope 10, the cover 20 can be disposed of after every surgery. Thus, unlike the case where a washing device that supplies a washing fluid to the observation window portion 14 is attached to the rigid endoscope 10, time and effort to wash such a washing device itself before every surgery can be omitted. Moreover, in the case of forming an endoscope unit by overlaying the cover 20 on the outer circumferential surface of the rigid endoscope 10, the cover 20 can be applied to a thinner rigid endoscope 10.

Since the material of the cover 20 is rubber, the cover 20 can have elasticity. Thus, the cover 20 can be brought into close contact with the outer circumferential surface of the rigid endoscope 10 when the cover 20 is overlaid on the rigid endoscope 10. Therefore, the washing fluids can be inhibited from leaking from the flow passages defined by the outer circumferential surface of the rigid endoscope 10 and the inner surfaces of the respective grooves 24 and 26. In particular, silicone rubber (especially, silicone rubber for medical use) is preferably used as the material of the cover 20. This is because silicone rubber is used in various medical fields, is inexpensive, and can ensure safety more reliably. In addition, Teflon rubber may be used as the material of the cover 20. Moreover, by bringing the cover 20 into close contact with the outer circumferential surface of the rigid endoscope 10, the cover 20 can be prevented from being detached from the rigid endoscope 10 while the endoscope unit is inserted into the body of a patient. Furthermore, in the case where the material of the cover 20 is rubber, action of overlaying the cover 20 on the rigid endoscope 10 and action of detaching the cover 20 from the rigid endoscope 10 can be easily performed. In addition, in the case where the material of the cover 20 is rubber, a tip portion of the rigid endoscope 10 can be covered with the cover 20 made of the rubber. Thus, even when the endoscope unit comes into contact with a site such as an organ within the body of a patient, the site is inhibited as much as possible from being damaged by the endoscope unit.

The material of the cover 20 which covers the entirety in the circumferential direction of the outer circumferential surface of the rigid endoscope 10 is not limited to rubber. A plastic material such as a soft plastic material may be used as the material of the cover 20. Even in this case, the endoscope unit in which the cover 20 is overlaid on the rigid endoscope 10 as shown in FIG. 3 is formed by putting the tip of the rigid endoscope 10 into the hollow portion at the base side (that is, the far side in FIG. 2) of the body portion 22 of the cover 20 and inserting the rigid endoscope 10 into the hollow portion of the cover 20 until the tip of the rigid endoscope 10 becomes caught on the fluid direction change portion 34 at the tip side of the cover 20.

In this example, the structure in which the cover 20 is overlaid on the rigid endoscope 10 in which the observation window portion 14 extends along a plane orthogonal to the longitudinal direction of the elongated substantially cylindrical body portion 12, has been described above. However, in an example, a rigid endoscope according to a modification, one having an observation window portion inclined relative to a plane orthogonal to the longitudinal direction of an elongated substantially cylindrical body portion may be used. Such a structure will be described with reference to FIG. 6. FIG. 6 is a cross-sectional view illustrating a state where a cover 20a is overlaid on a rigid endoscope 10a.

As shown in FIG. 6, the rigid endoscope 10a has an elongated substantially cylindrical body portion 12a, and an objective lens (not shown) for taking an image of an affected area and an observation window portion 14a such as a glass plate that protects the objective lens are provided at a tip of the body portion 12a. In addition, as described above, the observation window portion 14a is inclined relative to a plane orthogonal to the longitudinal direction of the elongated substantially cylindrical body portion 12a (a plane extending in the up-down direction in FIG. 6).

The cover 20a has an elongated substantially cylindrical body portion 22a, and is overlaid on the outer circumferential surface of the rigid endoscope 10a by inserting a tip of the rigid endoscope 10a into a hollow portion at the base side (that is, the right side in FIG. 6) of the body portion 22a. A tip portion of the cover 20a is also inclined relative to the plane orthogonal to the longitudinal direction of the body portion 22a so as to match the shape of the rigid endoscope 10a. In addition, two grooves 24a and 26a are formed on the inner circumferential surface of the body portion 22a of the cover 20a so as to extend along the longitudinal direction of the body portion 22a, and flow passages for washing fluids to be supplied to the observation window portion 14a of the rigid endoscope 10a are defined by the outer circumferential surface of the body portion 12a of the rigid endoscope 10a and the inner surfaces of the respective grooves 24a and 26a. Here, of the two grooves 24a and 26a, the groove 24a is used as a flow passage for washing water such as physiological saline to be supplied to the observation window portion 14a of the rigid endoscope 10a, and the other groove 26a is used as a flow passage for gas for blowing away the washing water adhering to the observation window portion 14a.

As shown in FIG. 6, an opening 32a is provided at a tip of the cover 20a, and the observation window portion 14a is exposed to the outside of the cover 20a by the opening 32a when the cover 20a is overlaid on the outer circumferential surface of the rigid endoscope 10a. In addition, a fluid direction change portion 34a that changes the direction of the washing fluids flowing along the respective grooves 24a and 26a (that is, along the longitudinal direction of the body portion 22a of the cover 20a) to a direction toward the observation window portion 14a, is provided near the opening 32a at the tip of the cover 20a. Specifically, the fluid direction change portion 34a is provided with a curved portion 36a that defines the flow passages for the washing fluids, and the direction of the washing fluids is changed by the curved portion 36a from the left direction to the up direction in FIG. 6. Since the direction of the washing fluids flowing in the left direction in FIG. 6 along the outer circumferential surface of the body portion 12a of the rigid endoscope 10a can be changed by such a fluid direction change portion 34a to the direction toward the observation window portion 14a of the rigid endoscope 10a, the observation window portion 14a can be reliably washed by the washing fluids.

As shown in FIG. 6, such a fluid direction change portion 34a is located in the vicinity of the edge, of the inclined observation window portion 14a, close to the base side of the body portion 12a (that is, the edge, of the observation window portion 14a, located at the rightmost side in FIG. 6). In other words, the fluid direction change portion 34a is provided at a location, on the inclined tip portion of the cover 20a, close to the base side of the body portion 22a. Therefore, the direction of the washing fluids flowing in the left direction in FIG. 6 along the outer circumferential surface of the body portion 12a of the rigid endoscope 10a can be more reliably changed to the direction toward the observation window portion 14a. The position of the fluid direction change portion 34a is not limited to the position shown in FIG. 6. The fluid direction change portion 34a may be provided at a location, on the tip portion of the cover 20a, distant from the base side of the body portion 22a, or may be provided at a middle part, of the tip portion of the cover 20a, between the location close to and the location distant from the base side of the body portion 22a. Moreover, fluid supply pipes 28 and 30 are respectively connected to the base end side of the respective grooves 24a and 26a in the body portion 22a of the cover 20a, and the washing water and the gas can be supplied from these fluid supply pipes 28 and 30 into the respective grooves 24a and 26a.

Even with the cover 20a and the rigid endoscope 10a according to the example shown in FIG. 6, the observation window portion 14a is allowed to be washed within the body of a patient, by providing the grooves 24a and 26a as the flow passages for the washing fluids on the inner circumferential surface of the cover 20a which covers the outer circumferential surface of the rigid endoscope 10a. Furthermore, since the grooves 24a and 26a are provided on the inner circumferential surface of the cover 20a, the outer diameter of the rigid endoscope 10a covered with the cover 20a (that is, the endoscope unit) can be made relatively small, whereby the surgery efficiency can be improved and the inside of the body of a patient can be inhibited from being damaged.

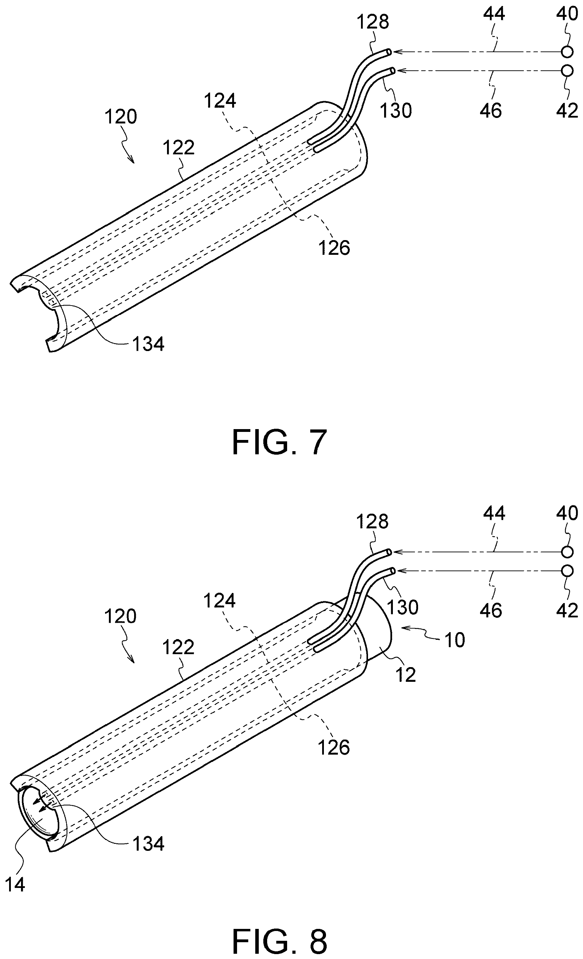

In an endoscope unit according to the invention, a cover that covers a part, not the entirety, in the circumferential direction of the outer circumferential surface of a rigid endoscope may be used. Such a structure will be described with reference to FIGS. 7 and 8. FIG. 7 is a perspective view illustrating the structure of a cover 120 according to the first embodiment of the present invention, and FIG. 8 is a perspective view illustrating a state where the cover 120 shown in FIG. 7 is overlaid on the rigid endoscope 10 shown in FIG. 1.

As shown in FIGS. 7 and 8, the cover 120 of the endoscope unit according to the invention has a substantially C-shape when the cover 120 is seen along the longitudinal direction thereof, and the rigid endoscope 10 is fitted into the space in the C-shaped portion. Such a cover 120 is formed from a plastic material such as a soft plastic material and has flexibility. Thus, in mounting the cover 120 onto the rigid endoscope 10, the gap of the C-shaped portion of the cover 120 is pressed against the outer circumferential surface of the rigid endoscope 10, and the cover 120 is pressed toward the rigid endoscope 10. By doing so, the cover 120 becomes deformed such that the gap of the C-shaped portion thereof is widened, thereby allowing the cover 120 to be mounted onto the rigid endoscope 10.

As shown in FIGS. 7 and 8, the cover 120 has an elongated body portion 122, and two grooves 124 and 126 are formed on the inner circumferential surface of the body portion 122 so as to extend along the longitudinal direction of the body portion 122. Flow passages for washing fluids to be supplied to the observation window portion 14 of the rigid endoscope 10 are defined by the outer circumferential surface of the body portion 12 of the rigid endoscope 10 and the inner surfaces of the respective grooves 124 and 126. Here, of the two grooves 124 and 126, the groove 124 is used as a flow passage for washing water such as physiological saline to be supplied to the observation window portion 14 of the rigid endoscope 10, and the other groove 126 is used as a flow passage for gas for blowing away the washing water adhering to the observation window portion 14. In addition, fluid supply pipes 128 and 130 are respectively connected to the base end side (that is, the far side in FIG. 7) of the respective grooves 124 and 126 in the body portion 122 of the cover 120, and the washing water and the gas can be supplied from these fluid supply pipes 128 and 130 into the respective grooves 124 and 126. Moreover, a fluid direction change portion 134 that changes the direction of the washing fluids flowing along the respective grooves 124 and 126 (that is, along the longitudinal direction of the body portion 122 of the cover 120) to a direction toward the observation window portion 14, is provided near a tip of the cover 120. Since the direction of the washing fluids flowing along the outer circumferential surface of the body portion 12 of the rigid endoscope 10 can be changed by such a fluid direction change portion 134 to the direction toward the observation window portion 14 of the rigid endoscope 10, the observation window portion 14 can be reliably washed by the washing fluids.

Even in the case where the cover 120 according to the embodiment shown in FIGS. 7 and 8 is used, the observation window portion 14 is allowed to be washed within the body of a patient, by providing the grooves 124 and 126 as the flow passages for the washing fluids on the inner circumferential surface of the cover 120 which covers a part in the circumferential direction of the outer circumferential surface of the rigid endoscope 10. Furthermore, since the grooves 124 and 126 are provided on the inner circumferential surface of the cover 120, the outer diameter of the rigid endoscope 10 covered with the cover 120 (that is, the endoscope unit) can be made relatively small, whereby the surgery efficiency can be improved and the inside of the body of a patient can be inhibited from being damaged.

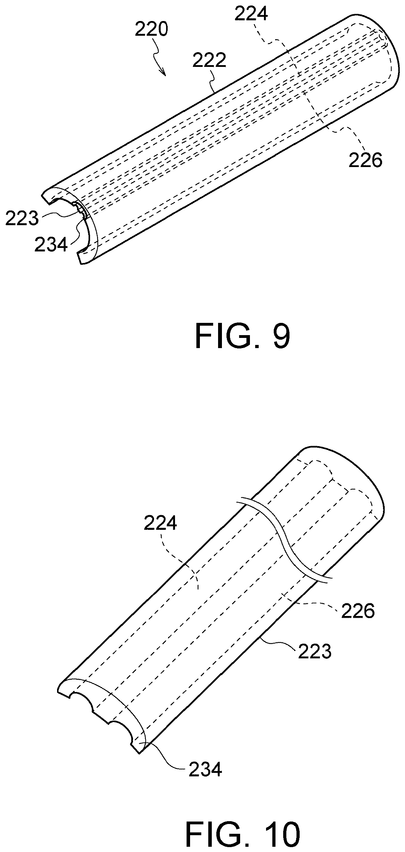

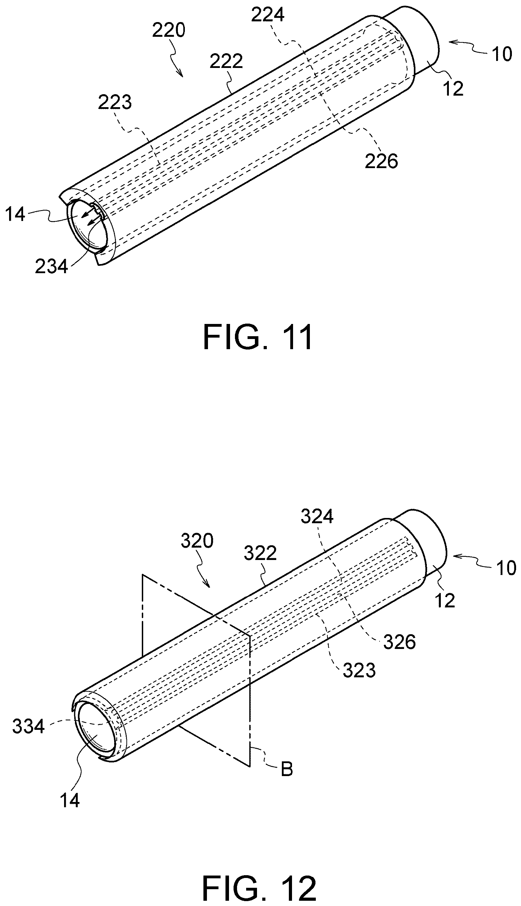

In an endoscope unit according to a modification, a cover, which covers a part in the circumferential direction of the outer circumferential surface of a rigid endoscope and which includes an outer cover portion and an inner cover portion, may be used. Such a structure will be described with reference to FIGS. 9 to 11. FIG. 9 is a perspective view illustrating the structure of a cover 220 according to a modification in the first embodiment of the present invention, FIG. 10 is a perspective view illustrating the detailed structure of an inner cover portion 223 of the cover 220 shown in FIG. 9, and FIG. 11 is a perspective view illustrating a state where the cover 220 shown in FIG. 9 is overlaid on the rigid endoscope 10 shown in FIG. 1.

As shown in FIGS. 9 and 11, the cover 220 of the endoscope unit according to the still another modification has a substantially C-shape when the cover 220 is seen along the longitudinal direction thereof, and the rigid endoscope 10 is fitted into the space in the C-shaped portion. Such a cover 220 has an outer cover portion 222 and the inner cover portion 223, and the outer cover portion 222 covers a part in the circumferential direction of the outer circumferential surface of the rigid endoscope 10. In addition, the outer cover portion 222 is formed from a material having flexibility (specifically, for example, a plastic material such as a soft plastic material), and thus the outer cover portion 222 bends when mounting the outer cover portion 222 onto the outer circumferential surface of the rigid endoscope 10. Moreover, the inner cover portion 223 is detachably mounted onto the inner surface of the outer cover portion 222, and two grooves 224 and 226 are formed on the inner circumferential surface of the inner cover portion 223. The detailed structure of such a cover 220 will be described below.

As described above, the outer cover portion 222 is formed from a plastic material such as a soft plastic material and has flexibility. Thus, in mounting the cover 220 onto the rigid endoscope 10, the gap of the C-shaped portion of the outer cover portion 222 is pressed against the outer circumferential surface of the rigid endoscope 10, and the outer cover portion 222 is pressed toward the rigid endoscope 10. By doing so, the outer cover portion 222 becomes deformed such that the gap of the C-shaped portion thereof is widened, thereby allowing the cover 220 to be mounted onto the rigid endoscope 10.

The inner cover portion 223 is detachably mounted onto the inner surface of the outer cover portion 222. Instead of the inner cover portion 223 being detachably mounted onto the inner surface of the outer cover portion 222, the inner cover portion 223 may be fixed to the inner surface of the outer cover portion 222 such that the inner cover portion 223 cannot be detached from the inner surface of the outer cover portion 222. The inner cover portion 223 is formed from rubber such as silicone rubber (especially, silicone rubber for medical use) or Teflon rubber. In addition, two grooves 224 and 226 are formed on the inner circumferential surface of the inner cover portion 223 so as to extend along the longitudinal direction of the inner cover portion 223. Flow passages for washing fluids to be supplied to the observation window portion 14 of the rigid endoscope 10 are defined by the outer circumferential surface of the body portion 12 of the rigid endoscope 10 and the inner surfaces of the respective grooves 224 and 226. Here, of the two grooves 224 and 226, the groove 224 is used as a flow passage for washing water such as physiological saline to be supplied to the observation window portion 14 of the rigid endoscope 10, and the other groove 226 is used as a flow passage for gas for blowing away the washing water adhering to the observation window portion 14. Moreover, fluid supply pipes (not shown) are respectively connected to the base end side (that is, the far side in FIGS. 9 and 10) of the respective grooves 224 and 226 in the inner cover portion 223, and the washing water and the gas can be supplied from these fluid supply pipes into the respective grooves 224 and 226. Furthermore, a fluid direction change portion 234 that changes the direction of the washing fluids flowing along the respective grooves 224 and 226 (that is, along the longitudinal direction of the inner cover portion 223) to a direction toward the observation window portion 14, is provided near a tip of the inner cover portion 223. Since the direction of the washing fluids flowing along the outer circumferential surface of the body portion 12 of the rigid endoscope 10 can be changed by such a fluid direction change portion 234 to the direction toward the observation window portion 14 of the rigid endoscope 10, the observation window portion 14 can be reliably washed by the washing fluids.

Even in the case where the cover 220 according to the modification shown in FIGS. 9 to 11 is used, the observation window portion 14 is allowed to be washed within the body of a patient, by providing the grooves 224 and 226 as the flow passages for the washing fluids on the inner circumferential surface of the inner cover portion 223 of the cover 220 which covers a part in the circumferential direction of the outer circumferential surface of the rigid endoscope 10. Furthermore, since the grooves 224 and 226 are provided on the inner circumferential surface of the inner cover portion 223 of the cover 220, the outer diameter of the rigid endoscope 10 covered with the cover 220 (that is, the endoscope unit) can be made relatively small, whereby the surgery efficiency can be improved and the inside of the body of a patient can be inhibited from being damaged. Moreover, the outer cover portion 222 has flexibility, and is mounted onto the outer circumferential surface of the rigid endoscope 10 by the outer cover portion 222 bending. Thus, the cover 220 can be easily mounted onto or detached from the outer circumferential surface of the rigid endoscope 10. Furthermore, since the flow passages for the washing fluids to be supplied to the observation window portion 14 of the rigid endoscope 10 are defined between the outer circumferential surface of the rigid endoscope 10 and the grooves 224 and 226 formed on the inner cover portion 223, the flow passages for the washing fluids can be easily formed as compared to the case where a supply passage for washing water and gas penetrates the inside of a washing sheath. Thus, the production cost of the cover 220 can be reduced.

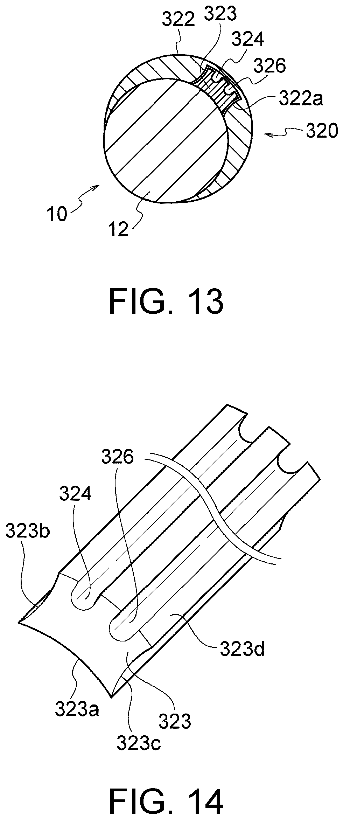

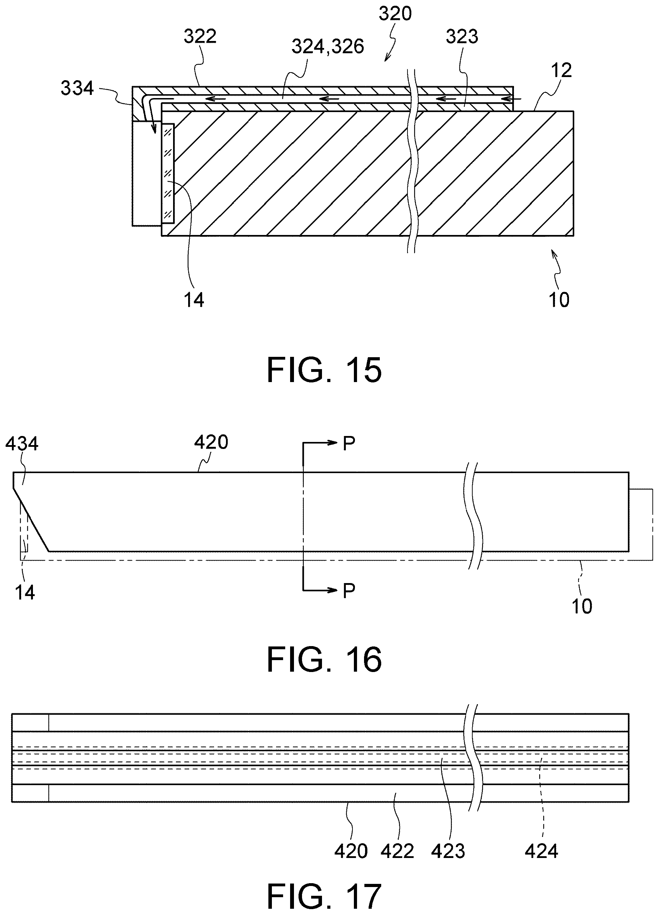

As an endoscope unit according to still another modification, one shown in FIGS. 12 to 15 may be used. In the endoscope unit shown in FIGS. 12 to 15 as well, similar to the endoscope unit shown in FIGS. 9 to 11, a cover covers a part in the circumferential direction of the outer circumferential surface of a rigid endoscope and includes an outer cover portion and an inner cover portion. In addition, in the endoscope unit shown in FIGS. 12 to 15, such a cover including the outer cover portion and the inner cover portion is overlaid on the rigid endoscope 10 shown in FIG. 1. Here, FIG. 12 is a perspective view illustrating the structure of the endoscope unit according to the still another modification in the first embodiment of the present invention, and FIG. 13 is a cross-sectional view of the endoscope unit shown in FIG. 12, taken along a virtual plane B. In addition, FIG. 14 is a perspective view illustrating the detailed structure of an inner cover portion 323 in a cover 320 of the endoscope unit shown in FIG. 12. Moreover, FIG. 15 is a cross-sectional view of the endoscope unit shown in FIG. 12, taken along a virtual plane passing through the center line of the rigid endoscope 10.

As shown in FIGS. 12 and 13, the cover 320 of the endoscope unit according to the still another modification has a substantially C-shape when the cover 320 is seen along the longitudinal direction thereof, and the rigid endoscope 10 is fitted into the space in the C-shaped portion. Such a cover 320 has an outer cover portion 322 and the inner cover portion 323, and the outer cover portion 322 covers a part in the circumferential direction of the outer circumferential surface of the rigid endoscope 10. In addition, the outer cover portion 322 is formed from a material having flexibility (specifically, for example, a plastic material such as a soft plastic material), and the outer cover portion 322 bends when mounting the outer cover portion 322 onto the outer circumferential surface of the rigid endoscope 10. Moreover, the inner cover portion 323 is detachably mounted onto the inner surface of the outer cover portion 322, and two grooves 324 and 326 are formed on the inner cover portion 323. The detailed structure of such a cover 320 will be described below.

As described above, the outer cover portion 322 is formed from a plastic material such as a soft plastic material and has flexibility. Thus, in mounting the cover 320 onto the rigid endoscope 10, the gap of the C-shaped portion of the outer cover portion 322 is pressed against the outer circumferential surface of the rigid endoscope 10, and the outer cover portion 322 is pressed toward the rigid endoscope 10. By doing so, the outer cover portion 322 becomes deformed such that the gap of the C-shaped portion thereof is widened, thereby allowing the cover 320 to be mounted onto the rigid endoscope 10.

The inner cover portion 323 is detachably mounted onto the inner surface of the outer cover portion 322. Specifically, as shown in FIG. 13, a recess 322a is formed on the inner surface of the outer cover portion 322. In addition, the inner cover portion 323 is detachably mounted to the recess 322a of the outer cover portion 322. Here, the inner cover portion 323 is formed from an elastically deformable material. More specifically, the inner cover portion 323 is formed from rubber such as silicone rubber (especially, silicone rubber for medical use) or Teflon rubber. The inner cover portion 323 has a shape that allows the inner cover portion 323 to be mounted to the recess 322a of the outer cover portion 322 by the inner cover portion 323 deforming elastically. More specifically, the inner cover portion 323 has a bottom portion 323a curved along the outer circumferential surface of the rigid endoscope 10, a pair of left and right side portions 323b and 323c, and an upper portion 323d having a shape along the bottom surface of the recess 322a of the outer cover portion 322, and the two grooves 324 and 326 are formed on the upper portion 323d. In addition, the side portions 323b and 323c each have a concave shape, and convex portions to which the concave-shaped portions of the respective side portions 323b and 323c are fitted are formed at side portions of the recess 322a of the outer cover portion 322. Therefore, the inner cover portion 323 becomes elastically deformed in mounting the inner cover portion 323 to the recess 322a of the outer cover portion 322. Moreover, when the inner cover portion 323 is mounted to the recess 322a of the outer cover portion 322, the convex portions formed at the side portions of the recess 322a of the outer cover portion 322 are fitted to the concave-shaped portions of the respective side portions 323b and 323c, whereby the inner cover portion 323 is prevented from being removed from the recess 322a of the outer cover portion 322. In the present embodiment, instead of the inner cover portion 323 being detachably mounted onto the inner surface of the outer cover portion 322, the inner cover portion 323 may be fixed to the inner surface of the outer cover portion 322 such that the inner cover portion 323 cannot be detached from the inner surface of the outer cover portion 322.

As described above, the two grooves 324 and 326 are formed on the upper portion 323d of the inner cover portion 323 so as to extend along the longitudinal direction of the inner cover portion 323. Flow passages for washing fluids to be supplied to the observation window portion 14 of the rigid endoscope 10 are defined between the recess 322a of the outer cover portion 322 and the inner surfaces of the respective grooves 324 and 326. Here, of the two grooves 324 and 326, the groove 324 is used as a flow passage for washing water such as physiological saline to be supplied to the observation window portion 14 of the rigid endoscope 10, and the other groove 326 is used as a flow passage for gas for blowing away the washing water adhering to the observation window portion 14. Moreover, fluid supply pipes (not shown) are respectively connected to the base end side (that is, the far side in FIGS. 12 and 14) of the respective grooves 324 and 326 in the inner cover portion 323, and the washing water and the gas can be supplied from these fluid supply pipes into the respective grooves 324 and 326. Furthermore, as shown in FIG. 15, a fluid direction change portion 334 that changes the direction of the washing fluids flowing along the respective grooves 324 and 326 (that is, along the longitudinal direction of the inner cover portion 323) to a direction toward the observation window portion 14, is provided at a tip portion of the outer cover portion 322. Since the direction of the washing fluids flowing in the longitudinal direction of the rigid endoscope 10 toward the tip of the rigid endoscope 10 can be changed by such a fluid direction change portion 334 to the direction toward the observation window portion 14 of the rigid endoscope 10, the observation window portion 14 can be reliably washed by the washing fluids.

Even in the case where the cover 320 according to the still another modification shown in FIGS. 12 to 15 is used, the observation window portion 14 is allowed to be washed within the body of a patient, by providing the grooves 324 and 326 as the flow passages for the washing fluids on the inner cover portion 323 of the cover 320 which covers a part in the circumferential direction of the outer circumferential surface of the rigid endoscope 10. Furthermore, the outer cover portion 322 has flexibility, and is mounted onto the outer circumferential surface of the rigid endoscope 10 by the outer cover portion 322 bending. Thus, the cover 320 can be easily mounted onto or detached from the outer circumferential surface of the rigid endoscope 10. Moreover, since the flow passages for the washing fluids to be supplied to the observation window portion 14 of the rigid endoscope 10 are defined between the inner surface of the outer cover portion 322 (specifically, the bottom surface of the recess 322a) and the grooves 324 and 326 formed on the inner cover portion 323, the flow passages for the washing fluids can be easily formed as compared to the case where a supply passage for washing water and gas penetrates the inside of a washing sheath. Thus, the production cost of the cover 320 can be reduced.

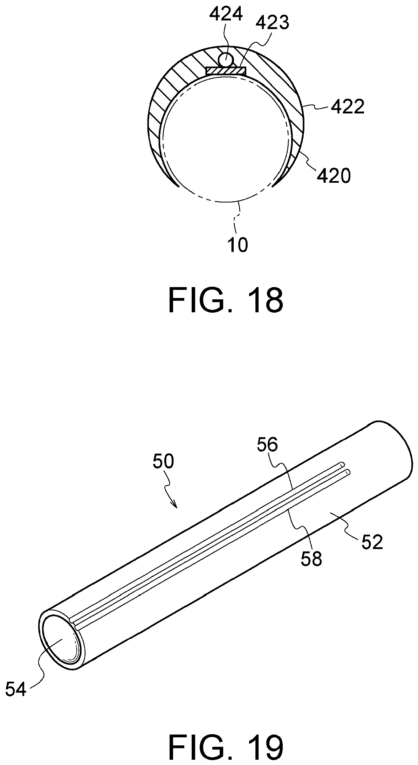

As an endoscope unit according to still another modification, one shown in FIGS. 16 to 18 may be used. In the endoscope unit shown in FIGS. 16 to 18 as well, similar to the endoscope unit shown in FIGS. 9 to 11 and the endoscope unit shown in FIGS. 12 to 15, a cover covers a part in the circumferential direction of the outer circumferential surface of a rigid endoscope and includes an outer cover portion and an inner cover portion. In addition, in the endoscope unit shown in FIGS. 16 to 18, such a cover including the outer cover portion and the inner cover portion is overlaid on the rigid endoscope 10 shown in FIG. 1. Here, FIG. 16 is a side view illustrating the structure of the cover of the endoscope unit according to the still another modification in the first embodiment of the present invention, and FIG. 17 is a bottom view illustrating the structure of the cover shown in FIG. 16, as seen from below. In addition, FIG. 18 is a cross-sectional view of the cover shown in FIG. 16, as seen from the direction of arrows P-P.

As shown in FIG. 18, the cover 420 of the endoscope unit according to the still another modification has a substantially C-shape when the cover 420 is seen along the longitudinal direction thereof, and the rigid endoscope 10 is fitted into the space in the C-shaped portion. Such a cover 420 has an outer cover portion 422 and the inner cover portion 423, and the outer cover portion 422 covers a part in the circumferential direction of the outer circumferential surface of the rigid endoscope 10. In addition, the outer cover portion 422 is formed from a material having flexibility (specifically, for example, a plastic material such as a soft plastic material), and the outer cover portion 422 bends when mounting the outer cover portion 422 onto the outer circumferential surface of the rigid endoscope 10. Moreover, the inner cover portion 423 is detachably mounted onto the inner surface of the outer cover portion 422. Furthermore, in the cover 420 shown in FIGS. 16 to 18, a flow passage 424 for washing fluids to be supplied to the observation window portion 14 of the rigid endoscope 10 (specifically, washing water such as physiological saline, and gas for blowing away the washing water adhering to the observation window portion 14) is defined between the outer cover portion 422 and the inner cover portion 423. The detailed structure of such a cover 420 will be described below.

As described above, the outer cover portion 422 is formed from a plastic material such as a soft plastic material and has flexibility. Thus, in mounting the cover 420 onto the rigid endoscope 10, the gap of the C-shaped portion of the outer cover portion 422 is pressed against the outer circumferential surface of the rigid endoscope 10, and the outer cover portion 422 is pressed toward the rigid endoscope 10. By doing so, the outer cover portion 422 becomes deformed such that the gap of the C-shaped portion thereof is widened, thereby allowing the cover 420 to be mounted onto the rigid endoscope 10.

The inner cover portion 423 is detachably mounted onto the inner surface of the outer cover portion 422. Specifically, as shown in FIG. 18, a recess is formed on the inner surface of the outer cover portion 422. In addition, the inner cover portion 423 is detachably mounted to the recess of the outer cover portion 422. Here, the inner cover portion 423 is formed from an elastically deformable material. More specifically, the inner cover portion 423 is formed from rubber such as silicone rubber (especially, silicone rubber for medical use) or Teflon rubber. The inner cover portion 423 has a shape that allows the inner cover portion 423 to be mounted to the recess of the outer cover portion 422 by the inner cover portion 423 deforming elastically. Specifically, in the cover 420 shown in FIGS. 16 to 18, the inner cover portion 423 is an elongated plate. In addition, when the inner cover portion 423 is mounted to the recess of the outer cover portion 422, the flow passage 424 for the washing fluids to be supplied to the observation window portion 14 of the rigid endoscope 10 is defined between the outer cover portion 422 and the inner cover portion 423. In the present embodiment, instead of the inner cover portion 423 being detachably mounted onto the inner surface of the outer cover portion 422, the inner cover portion 423 may be fixed to the inner surface of the outer cover portion 422 such that the inner cover portion 423 cannot be detached from the inner surface of the outer cover portion 422.

As described above, the flow passage 424 for the washing fluids to be supplied to the observation window portion 14 of the rigid endoscope 10 is defined between the outer cover portion 422 and the inner cover portion 423 when the inner cover portion 423 is mounted to the recess of the outer cover portion 422. The flow passage 424 is formed so as to extend along the longitudinal direction of the inner cover portion 423. In addition, a fluid supply pipe (not shown) is connected to the base end side (that is, the right side in FIGS. 16 and 17) of the flow passage 424, and the washing water and the gas can be supplied from this fluid supply pipe to the flow passage 424 of the cover 420. Furthermore, as shown in FIG. 16, a fluid direction change portion 434 that changes the direction of the washing fluids flowing along the flow passage 424 (that is, along the longitudinal direction of the inner cover portion 423) to a direction toward the observation window portion 14, is provided at a tip portion of the outer cover portion 422. Such a fluid direction change portion 434 has substantially the same structure as the above-described fluid direction change portion 334. Since the direction of the washing fluids flowing in the longitudinal direction of the rigid endoscope 10 toward the tip of the rigid endoscope 10 can be changed by such a fluid direction change portion 434 to the direction toward the observation window portion 14, the observation window portion 14 can be reliably washed by the washing fluids.

Even in the case where the cover 420 according to the still another modification shown in FIGS. 16 to 18 is used, the observation window portion 14 can be washed within the body of a patient, since the flow passage 424 for the washing fluids is defined by the inner cover portion 423 of the cover 420 which covers a part in the circumferential direction of the outer circumferential surface of the rigid endoscope 10. Furthermore, the outer cover portion 422 has flexibility, and is mounted onto the outer circumferential surface of the rigid endoscope 10 by the outer cover portion 422 bending. Thus, the cover 420 can be easily mounted onto or detached from the outer circumferential surface of the rigid endoscope 10. Moreover, since the flow passage 424 for the washing fluids to be supplied to the observation window portion 14 of the rigid endoscope 10 is defined between the inner surface of the outer cover portion 422 (specifically, the bottom surface of the recess) and the inner cover portion 423, the flow passage for the washing fluids can be easily formed as compared to the case where a supply passage for washing water and gas penetrates the inside of a washing sheath. Thus, the production cost of the cover 420 can be reduced. Furthermore, in the cover 420 shown in FIGS. 16 to 18, the inner cover portion 423 is an elongated plate, and the cover 420 is formed by fitting the plate-like inner cover portion 423 into the recess of the outer cover portion 422. Thus, such an elongated plate-like inner cover portion 423 is easily produced.

In the endoscope unit shown in FIGS. 9 to 11, the endoscope unit shown in FIGS. 12 to 15, or the endoscope unit shown in FIGS. 16 to 18, a method in which the cover 220, 320, or 420 is composed of only the outer cover portion 222, 322, or 422 having flexibility is also conceivable. Specifically, a method in which the inner cover portion 223, 323, or 423 is not provided to the cover 220, 320, or 420 is also conceivable. However, in this case, flow passages for the washing fluids to be supplied to the observation window portion 14 of the rigid endoscope 10 (specifically, for example, grooves) have to be formed on the outer cover portion 222, 322, or 422. Here, the outer cover portion 222, 322, or 422 is generally produced by molding. In the molding, it is difficult to form flow passages for the washing fluids (specifically, for example, grooves) extending over the entire region in the longitudinal direction of the cover 220, 320, or 420, on the outer cover portion 222, 322, or 422 having a substantially C cross-sectional shape. On the other hand, in the case of providing the inner cover portion 223, 323, or 423 on the inner surface of the outer cover portion 222, 322, or 422, the outer cover portion 222, 322, or 422 can be easily molded.

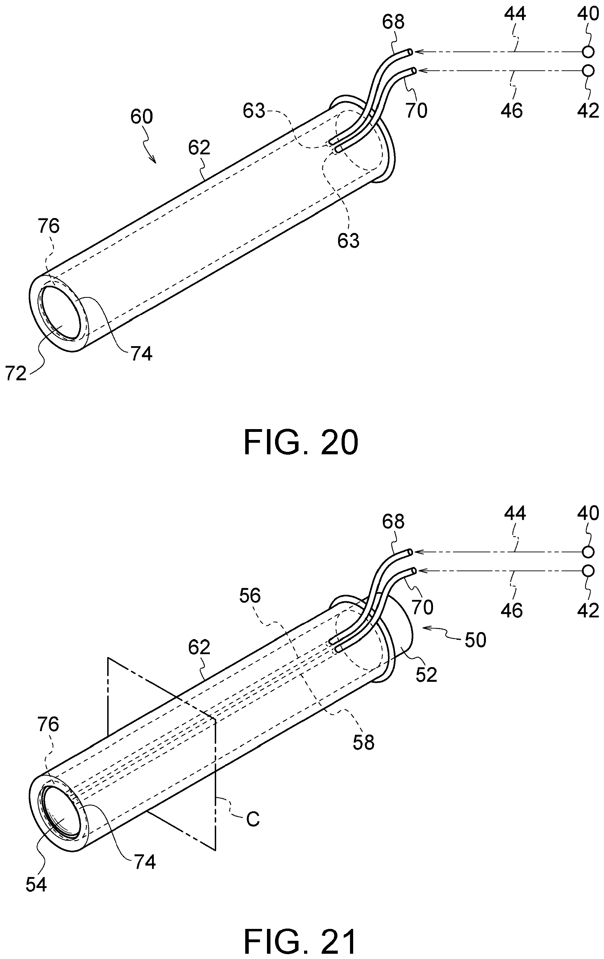

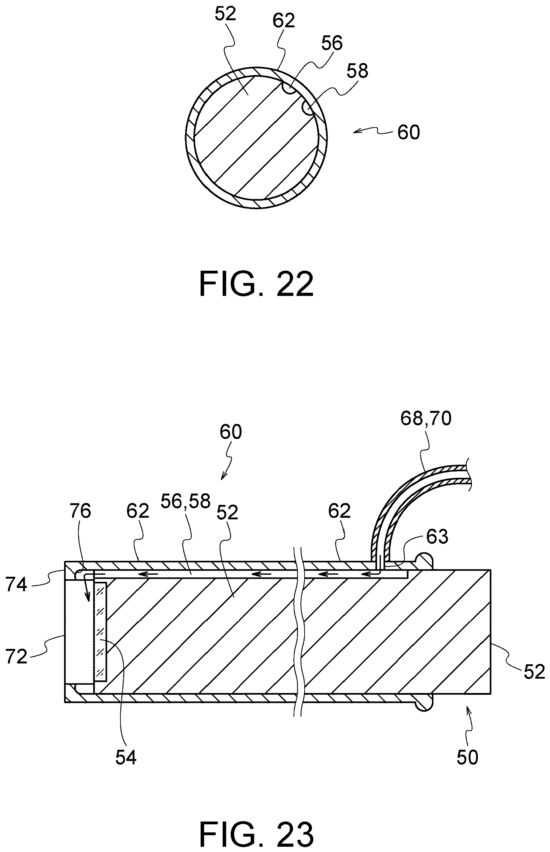

Next, a second example will be described with reference to the drawings. FIGS. 19 to 23 illustrate an endoscope unit according to the second example. Among these figures, FIG. 19 is a perspective view schematically illustrating the structure of a rigid endoscope in the endoscope unit, and FIG. 20 is a perspective view illustrating the structure of a rigid endoscope cover. In addition, FIG. 21 is a perspective view illustrating a state where the cover shown in FIG. 20 is overlaid on the rigid endoscope shown in FIG. 19. Moreover, FIG. 22 is a cross-sectional view of the endoscope unit shown in FIG. 21, taken along a virtual plane C, and FIG. 23 is a cross-sectional view of the endoscope unit shown in FIG. 21, taken along a virtual plane passing through the center line of the rigid endoscope.

First, the rigid endoscope 50 will be described with reference to FIG. 19. As shown in FIG. 19, the rigid endoscope 50 has an elongated substantially cylindrical body portion 52, and an objective lens (not shown) for taking an image of an affected area and an observation window portion 54 such as a glass plate that protects the objective lens are provided at a tip of the body portion 52. In addition, two grooves 56 and 58 are formed on the outer circumferential surface of the body portion 52 so as to extend substantially parallel to each other along the longitudinal direction of the body portion 52.

Next, a hollow cover 60 for covering the outer circumferential surface of the rigid endoscope 50 will be described with reference to FIGS. 20 to 23. The material of the cover 60 is rubber such as natural rubber or synthetic rubber, and the cover 60 is overlaid on the outer circumferential surface of the rigid endoscope 50 shown in FIG. 19. More specifically, the cover 60 has an elongated substantially cylindrical body portion 62, and is overlaid on the outer circumferential surface of the rigid endoscope 50 by inserting a tip of the rigid endoscope 50 into a hollow portion at the base side (that is, the far side in FIG. 20) of the body portion 62. Unlike the cover 20 shown in FIG. 2, etc., no groove is provided on the inner circumferential surface of the cover 60. The endoscope unit is formed by combining the rigid endoscope 50 and the cover 60 described above. In addition, when the cover 60 is overlaid on the outer circumferential surface of the rigid endoscope 50, flow passages for washing fluids to be supplied to the observation window portion 54 of the rigid endoscope 50 are defined by the inner circumferential surface of the cover 60 and the inner surfaces of the respective grooves 56 and 58 of the rigid endoscope 50. Here, of the two grooves 56 and 58, the groove 56 is used as a flow passage for washing water such as physiological saline to be supplied to the observation window portion 54 of the rigid endoscope 50, and the other groove 58 is used as a flow passage for gas for blowing away the washing water adhering to the observation window portion 54.

As described above, unlike the cover 20 according to the first example shown in FIG. 2, etc., no groove is provided on the inner circumferential surface of the cover 60. Thus, the thickness of the cover 60 can be made smaller than the thickness of the cover 20 according to the first example. Therefore, when the diameter of the rigid endoscope 10 according to the first example and the diameter of the rigid endoscope 50 according to the second example are substantially equal to each other, the diameter of the endoscope unit according to the second example can be made smaller than the diameter of the endoscope unit according to the first example.

As shown in FIG. 20, an opening 72 is provided at a tip of the cover 60, and the observation window portion 54 is exposed to the outside of the cover 60 by the opening 72 when the cover 60 is overlaid on the outer circumferential surface of the rigid endoscope 50. In addition, as shown in FIG. 23, a fluid direction change portion 74 that changes the direction of the washing fluids flowing along the respective grooves 56 and 58 (that is, along the longitudinal direction of the body portion 52 of the rigid endoscope 50) to a direction toward the observation window portion 54 (that is, a direction toward the hollow portion of the cover 60), is provided at the tip of the cover 60. Specifically, the fluid direction change portion 74 is provided with a curved portion 76 that defines the flow passages for the washing fluids, and the direction of the washing fluids is changed by the curved portion 76 from the left direction to a downward right direction in FIG. 23. Such a fluid direction change portion 74 is provided over the entire circumference of the tip of the cover 60. Since the direction of the washing fluids flowing in the left direction in FIG. 23 along the respective grooves 56 and 58 formed on the body portion 52 of the rigid endoscope 50 can be changed by such a fluid direction change portion 74 to the direction toward the observation window portion 54 of the rigid endoscope 50, the observation window portion 54 can be reliably washed by the washing fluids. Moreover, such a fluid direction change portion 74 projects inward from the body portion 62 toward the hollow portion of the cover 60, and is formed such that the tip of the rigid endoscope 50 is caught on the fluid direction change portion 74 when the cover 60 is overlaid on the rigid endoscope 50. Therefore, the tip of the rigid endoscope 50 can be prevented from protruding from the tip of the cover 60.

As shown in FIGS. 21 and 23, fluid supply pipes 68 and 70 are connected to the base end side (that is, the far side in FIG. 21) of the body portion 62 of the cover 60, and through holes 63 (see FIG. 23) are formed in the body portion 62 of the cover 60 at locations to which the respective fluid supply pipes 68 and 70 are attached. Therefore, when the cover 60 is overlaid on the rigid endoscope 50, the washing water and the gas can be supplied from these fluid supply pipes 68 and 70 through the through holes 63 into the respective grooves 56 and 58 of the rigid endoscope 50. In addition, as shown in FIG. 20, a washing water supply source 40 is connected to the fluid supply pipe 68 via a washing water supply passage 44, and the washing water supplied from the washing water supply source 40 to the washing water supply passage 44 is sent to the fluid supply pipe 68. Therefore, when the cover 60 is overlaid on the rigid endoscope 50, the washing water can be supplied from the fluid supply pipe 68 to the flow passage defined between the inner circumferential surface of the body portion 62 of the cover 60 and the inner surface of the groove 56 of the rigid endoscope 50. Moreover, as shown in FIG. 20, a gas supply source 42 is connected to the fluid supply pipe 70 via a gas supply passage 46, and the gas supplied from the gas supply source 42 to the gas supply passage 46 is sent to the fluid supply pipe 70. Therefore, when the cover 60 is overlaid on the rigid endoscope 50, the gas can be supplied from the fluid supply pipe 70 to the flow passage defined between the inner circumferential surface of the body portion 62 of the cover 60 and the inner surface of the groove 58 of the rigid endoscope 50.

Next, a method for using the endoscope unit having such a structure will be described. First, in performing surgery in which the endoscope unit is inserted into the body of a patient, the cover 60 shown in FIG. 20 is overlaid on the outer circumferential surface of the rigid endoscope 50 shown in FIG. 19. Specifically, the tip of the rigid endoscope 50 is put into the hollow portion at the base side (that is, the far side in FIG. 20) of the body portion 62 of the cover 60, and the rigid endoscope 50 is inserted into the hollow portion of the cover 60 until the tip of the rigid endoscope 50 becomes caught on the fluid direction change portion 74 at the tip side of the cover 60. At this time, the positions of the respective through holes 63, which are provided in the body portion 62 of the cover 60 at the locations to which the respective fluid supply pipes 68 and 70 are attached, are caused to substantially coincide with the positions of the respective grooves 56 and 58 provided on the outer circumferential surface of the body portion 52 of the rigid endoscope 50. By doing so, the endoscope unit in which the cover 60 is overlaid on the rigid endoscope 50 as shown in FIG. 21 is formed. Then, the endoscope unit shown in FIG. 21 is inserted into the body of the patient, and the surgery is performed.

When the observation window portion 54 of the rigid endoscope 50 becomes dirty with blood, fat, etc., or becomes cloudy during the surgery, the observation window portion 54 is washed in a state where the endoscope unit has been inserted into the body of the patient. More specifically, by supplying the washing water from the washing water supply source 40 through the washing water supply passage 44 to the fluid supply pipe 68, the washing water flows in the left direction in FIG. 23 in the flow passage defined between the inner circumferential surface of the body portion 62 of the cover 60 and the inner surface of the groove 56 of the rigid endoscope 50. Then, the direction of the washing water flowing in the left direction in FIG. 23 as described above is changed at the tip of the cover 60 by the fluid direction change portion 74 to the direction toward the observation window portion 54. More specifically, the direction of the washing water flowing in the left direction in FIG. 23 along the flow passage defined between the inner circumferential surface of the body portion 62 of the cover 60 and the inner surface of the groove 56 of the rigid endoscope 50 is changed by the curved portion 76 of the fluid direction change portion 74 from the left direction to the downward right direction in FIG. 23. Therefore, the observation window portion 54 can be washed by the washing water. Thereafter, by supplying the gas from the gas supply source 42 through the gas supply passage 46 to the fluid supply pipe 70, the gas flows in the left direction in FIG. 23 in the flow passage defined between the inner circumferential surface of the body portion 62 of the cover 60 and the inner surface of the groove 58 of the rigid endoscope 50. Then, the direction of the gas flowing in the left direction in FIG. 23 as described above is changed at the tip of the cover 60 by the fluid direction change portion 74 to the direction toward the observation window portion 54. More specifically, the direction of the gas flowing in the left direction in FIG. 23 along the flow passage defined between the inner circumferential surface of the body portion 62 of the cover 60 and the inner surface of the groove 56 of the rigid endoscope 50 is changed by the curved portion 76 of the fluid direction change portion 74 from the left direction to the downward right direction in FIG. 23. Therefore, the washing water adhering to the observation window portion 54 can be blown away by the gas.

In the endoscope unit of the present embodiment having the structure as described above, the grooves 56 and 58 are provided on the outer circumferential surface of the rigid endoscope 50, and the flow passages for the washing fluids such as the washing water and the gas to be supplied to the observation window portion 54 of the rigid endoscope 50 are defined by the inner circumferential surface of the cover 60 and the inner surfaces of the respective grooves 56 and 58. The observation window portion 54 is allowed to be washed within the body of a patient, by providing the grooves 56 and 58 as the flow passages for the washing fluids on the outer circumferential surface of the rigid endoscope 50 as described above. Furthermore, since the grooves 56 and 58 are provided on the outer circumferential surface of the rigid endoscope 50, the outer diameter of the rigid endoscope 50 covered with the cover 60 (that is, the endoscope unit) can be made relatively small, whereby the surgery efficiency can be improved and the inside of the body of a patient can be inhibited from being damaged.

Since such a cover 60 is merely overlaid on the rigid endoscope 50, the cover 60 can be disposed of after every surgery. Thus, unlike the case where a washing device that supplies a washing fluid to the observation window portion 54 is attached to the rigid endoscope 50, time and effort to wash such a washing device itself before every surgery can be omitted. Moreover, in the case of forming an endoscope unit by overlaying the cover 60 on the outer circumferential surface of the rigid endoscope 50, the cover 60 can be applied to a thinner rigid endoscope 50.

Since the material of the cover 60 is rubber, the cover 60 can have elasticity. Thus, the cover 60 can be brought into close contact with the outer circumferential surface of the rigid endoscope 50 when the cover 60 is overlaid on the rigid endoscope 50. Therefore, the washing fluids can be inhibited from leaking from the flow passages defined by the inner circumferential surface of the cover 60 and the inner surfaces of the respective grooves 56 and 58. In particular, silicone rubber (especially, silicone rubber for medical use) is preferably used as the material of the cover 60. This is because silicone rubber is used in various medical fields, is inexpensive, and can ensure safety more reliably. In addition, Teflon rubber may be used as the material of the cover 60. Moreover, by bringing the cover 60 into close contact with the outer circumferential surface of the rigid endoscope 50, the cover 60 can be prevented from being detached from the rigid endoscope 50 while the endoscope unit is inserted into the body of a patient. Furthermore, in the case where the material of the cover 60 is rubber, action of overlaying the cover 60 on the rigid endoscope 50 and action of detaching the cover 60 from the rigid endoscope 50 can be easily performed. In addition, in the case where the material of the cover 60 is rubber, a tip portion of the rigid endoscope 50 can be covered with the cover 60 made of the rubber. Thus, even when the endoscope unit comes into contact with a site such as an organ within the body of a patient, the site is inhibited as much as possible from being damaged by the endoscope unit.

The material of the cover 60 which covers the entirety in the circumferential direction of the outer circumferential surface of the rigid endoscope 50 is not limited to rubber. A plastic material such as a soft plastic material may be used as the material of the cover 60. Even in this case, the endoscope unit in which the cover 60 is overlaid on the rigid endoscope 50 as shown in FIG. 21 is formed by putting the tip of the rigid endoscope 50 into the hollow portion at the base side (that is, the far side in FIG. 20) of the body portion 62 of the cover 60 and inserting the rigid endoscope 50 into the hollow portion of the cover 60 until the tip of the rigid endoscope 50 becomes caught on the fluid direction change portion 74 at the tip side of the cover 60.

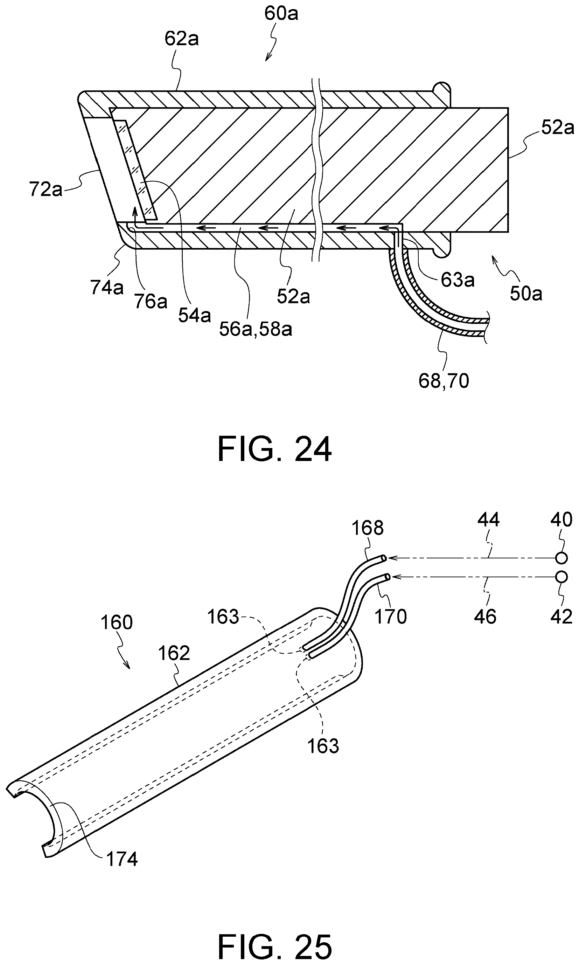

In the second example of the present disclosure, the structure in which the cover 60 is overlaid on the rigid endoscope 50 in which the observation window portion 54 extends along a plane orthogonal to the longitudinal direction of the elongated substantially cylindrical body portion 52, has been described above. Optionally, a rigid endoscope having an observation window portion inclined relative to a plane orthogonal to the longitudinal direction of an elongated substantially cylindrical body portion may be used. Such a structure will be described with reference to FIG. 24. FIG. 24 is a cross-sectional view illustrating a state where a cover 60a is overlaid on a rigid endoscope 50a.

As shown in FIG. 24, the rigid endoscope 50a has an elongated substantially cylindrical body portion 52a, and an objective lens (not shown) for taking an image of an affected area and an observation window portion 54a such as a glass plate that protects the objective lens are provided at a tip of the body portion 52a. In addition, two grooves 56a and 58a are formed on the outer circumferential surface of the body portion 52a so as to extend substantially parallel to each other along the longitudinal direction of the body portion 52a. Here, the respective grooves 56a and 58a extend from a location, on an inclined tip portion of the rigid endoscope 50a, close to the base side of the body portion 52a (that is, the lower end in FIG. 24) toward the base side of the body portion 52a.

The cover 60a has an elongated substantially cylindrical body portion 62a, and is overlaid on the outer circumferential surface of the rigid endoscope 50a by inserting a tip of the rigid endoscope 50a into a hollow portion at the base side (that is, the right side in FIG. 24) of the body portion 62a. No groove is provided on the inner circumferential surface of the cover 60a. In addition, when the cover 60a is overlaid on the outer circumferential surface of the rigid endoscope 50a, flow passages for washing fluids to be supplied to the observation window portion 54a of the rigid endoscope 50a are defined by the inner circumferential surface of the cover 60a and the inner surfaces of the respective grooves 56a and 58a of the rigid endoscope 50a. Here, of the two grooves 56a and 58a, the groove 56a is used as a flow passage for washing water such as physiological saline to be supplied to the observation window portion 54a of the rigid endoscope 50a, and the other groove 58a is used as a flow passage for gas for blowing away the washing water adhering to the observation window portion 54a.

As shown in FIG. 24, an opening 72a is provided at a tip of the cover 60a, and the observation window portion 54a is exposed to the outside of the cover 60a by the opening 72a when the cover 60a is overlaid on the outer circumferential surface of the rigid endoscope 50a. In addition, as shown in FIG. 24, a fluid direction change portion 74a that changes the direction of the washing fluids flowing along the respective grooves 56a and 58a (that is, along the longitudinal direction of the body portion 52a of the rigid endoscope 50a) to a direction toward the observation window portion 54a (that is, a direction toward the hollow portion of the cover 60a), is provided at the tip of the cover 60a. Specifically, the fluid direction change portion 74a is provided with a curved portion 76a that defines the flow passages for the washing fluids, and the direction of the washing fluids is changed by the curved portion 76a from the left direction to the up direction in FIG. 24. Since the direction of the washing fluids flowing in the left direction in FIG. 24 along the respective grooves 56a and 58a formed on the body portion 52a of the rigid endoscope 50a can be changed by such a fluid direction change portion 74a to the direction toward the observation window portion 54a of the rigid endoscope 50a, the observation window portion 54a can be reliably washed by the washing fluids.

As shown in FIG. 24, such a fluid direction change portion 74a is located in the vicinity of the edge, of the inclined observation window portion 54a, close to the base side of the body portion 52a (that is, the edge located at the rightmost side in FIG. 24). In other words, the fluid direction change portion 74a is provided at a location, on the inclined tip portion of the cover 60a, close to the base side of the body portion 62a. Therefore, the direction of the washing fluids flowing in the left direction in FIG. 24 along the respective grooves 56a and 58a formed on the body portion 52a of the rigid endoscope 50a can be more reliably changed to the direction toward the observation window portion 54a. Fluid supply pipes 68 and 70 are connected to the base end side of the body portion 62a of the cover 60a, and through holes 63a are formed in the body portion 62a of the cover 60a at locations to which the respective fluid supply pipes 68 and 70 are attached. Therefore, when the cover 60a is overlaid on the rigid endoscope 50a, the washing water and the gas can be supplied from these fluid supply pipes 68 and 70 through the through holes 63a into the respective grooves 56a and 58a of the rigid endoscope 50a.

The respective grooves 56a and 58a are not limited to grooves extending from the location, on the inclined tip portion of the rigid endoscope 50a, close to the base side of the body portion 52a (that is, the lower end in FIG. 24) toward the base side of the body portion 52a. In another example, the respective grooves 56a and 58a may extend from a location, on the inclined tip portion of the rigid endoscope 50a, distant from the base side of the body portion 52a (that is, the upper end in FIG. 24) toward the base side of the body portion 52a. In still another example, the respective grooves 56a and 58a may extend from a location, on the inclined tip portion of the rigid endoscope 50a, between the location close to the base side of the body portion 52a and the location distant from the base side toward the base side of the body portion 52a.

Even with the cover 60a and the rigid endoscope 50a according to the modification shown in FIG. 24, the observation window portion 54a is allowed to be washed within the body of a patient, by providing the grooves 56a and 58a as the flow passages for the washing fluids on the outer circumferential surface of the rigid endoscope 50a. Furthermore, since the grooves 56a and 58a are provided on the outer circumferential surface of the rigid endoscope 50a, the outer diameter of the rigid endoscope 50a covered with the cover 60a (that is, the endoscope unit) can be made relatively small, whereby the surgery efficiency can be improved and the inside of the body of a patient can be inhibited from being damaged.

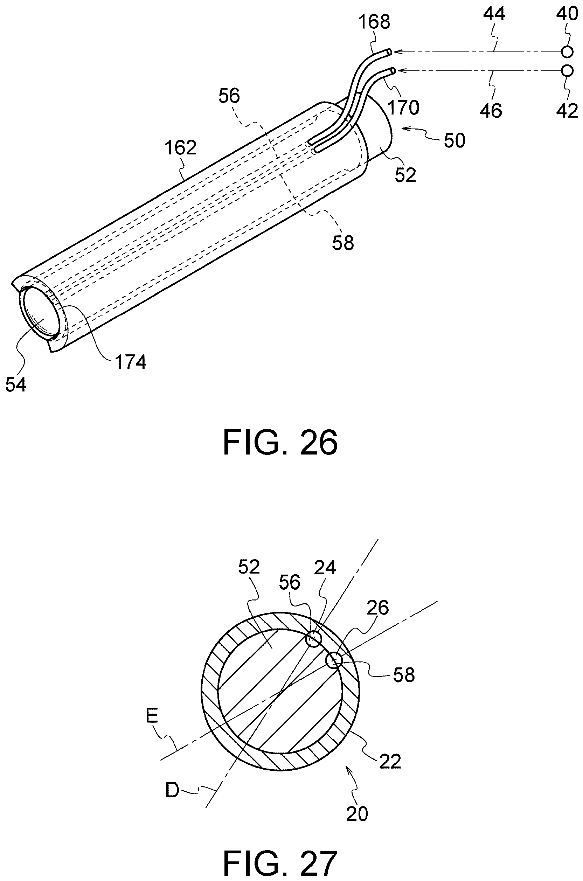

In an endoscope unit according to another modification, a cover that covers a part, not the entirety, in the circumferential direction of the outer circumferential surface of a rigid endoscope may be used. Such a structure will be described with reference to FIGS. 25 and 26. FIG. 25 is a perspective view illustrating the structure of a cover 160 according to the other modification in the second example of the present disclosure, and FIG. 26 is a perspective view illustrating a state where the cover 160 shown in FIG. 25 is overlaid on the rigid endoscope 50 shown in FIG. 19.

As shown in FIGS. 25 and 26, the cover 160 of the endoscope unit according to the other modification has a substantially C-shape when the cover 160 is seen along the longitudinal direction thereof, and the rigid endoscope 50 is fitted into the space in the C-shaped portion. Such a cover 160 is formed from a plastic material such as a soft plastic material and has flexibility. Thus, in mounting the cover 160 onto the rigid endoscope 50, the gap of the C-shaped portion of the cover 160 is pressed against the outer circumferential surface of the rigid endoscope 50, and the cover 160 is pressed toward the rigid endoscope 50. By doing so, the cover 160 becomes deformed such that the gap of the C-shaped portion thereof is widened, thereby allowing the cover 160 to be mounted onto the rigid endoscope 50.

As shown in FIGS. 25 and 26, the cover 160 has an elongated body portion 162, but no groove is provided on the inner circumferential surface of the cover 160. In addition, when the cover 160 is overlaid on the outer circumferential surface of the rigid endoscope 50, flow passages for washing fluids to be supplied to the observation window portion 54 of the rigid endoscope 50 are defined by the inner circumferential surface of the cover 160 and the inner surfaces of the respective grooves 56 and 58 of the rigid endoscope 50. Here, of the two grooves 56 and 58, the groove 56 is used as a flow passage for washing water such as physiological saline to be supplied to the observation window portion 54 of the rigid endoscope 50, and the other groove 58 is used as a flow passage for gas for blowing away the washing water adhering to the observation window portion 54. In addition, fluid supply pipes 168 and 170 are connected to the base end side (that is, the far side in FIG. 25) of the body portion 162 of the cover 160, and through holes 163 are formed in the body portion 162 of the cover 160 at locations to which the respective fluid supply pipes 168 and 170 are attached. Therefore, when the cover 160 is overlaid on the rigid endoscope 50, the washing water and the gas can be supplied from these fluid supply pipes 168 and 170 through the through holes 163 into the respective grooves 56 and 58 of the rigid endoscope 50. Moreover, a fluid direction change portion 174 that changes the direction of the washing fluids flowing along the respective grooves 56 and 58 (that is, along the longitudinal direction of the body portion 52 of the rigid endoscope 50) to a direction toward the observation window portion 54, is provided near a tip of the cover 160. Since the direction of the washing fluids flowing along the outer circumferential surface of the body portion 52 of the rigid endoscope 50 can be changed by such a fluid direction change portion 174 to the direction toward the observation window portion 54 of the rigid endoscope 50, the observation window portion 54 can be reliably washed by the washing fluids.

Even in the case where the cover 160 according to the other modification shown in FIGS. 25 and 26 is used, the observation window portion 54 can be washed within the body of a patient. Furthermore, since the grooves 56 and 58 are provided on the outer circumferential surface of the rigid endoscope 50, the outer diameter of the rigid endoscope 50 covered with the cover 160 (that is, the endoscope unit) can be made relatively small, whereby the surgery efficiency can be improved and the inside of the body of a patient can be inhibited from being damaged.

The endoscope unit according to an embodiment of present invention is not limited to the above-described structures, and various modifications can be made thereto.

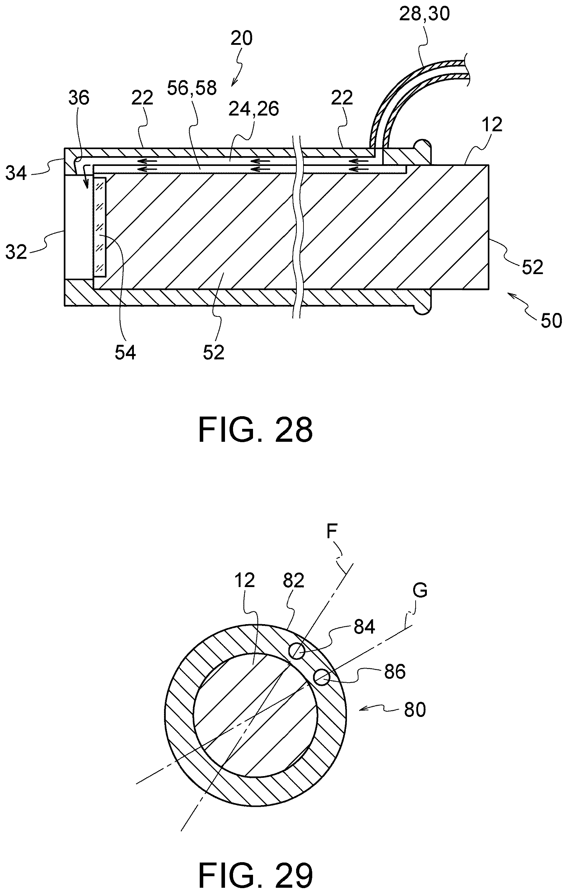

For example, an endoscope unit may be formed by overlaying the cover 20 shown in FIG. 2 on the rigid endoscope 50 shown in FIG. 19. Such an endoscope unit will be described with reference to FIGS. 27 and 28. FIG. 27 is a cross-sectional view of an endoscope unit in which the cover 20 shown in FIG. 2 is overlaid on the rigid endoscope 50 shown in FIG. 19, and FIG. 28 is a cross-sectional view of the endoscope unit shown in FIG. 27, taken along a virtual plane D or a virtual plane E passing through the center line of the rigid endoscope 50.

As shown in FIG. 27, in the case of forming an endoscope unit by overlaying the cover 20 shown in FIG. 2 on the rigid endoscope 50 shown in FIG. 19, in overlaying the cover 20 on the outer circumferential surface of the rigid endoscope 50, the cover 20 is aligned with the rigid endoscope 50 such that the positions of the respective grooves 56 and 58, which are provided on the outer circumferential surface of the body portion 52 of the rigid endoscope 50, substantially coincide with the positions of the respective grooves 24 and 26 provided on the inner circumferential surface of the body portion 22 of the cover 20. In such an endoscope unit, as shown in FIG. 28, flow passages for washing fluids such as washing water and gas to be supplied to the observation window portion 54 of the rigid endoscope 50 are defined by the inner surfaces of the respective grooves 56 and 58 provided on the outer circumferential surface of the body portion 52 of the rigid endoscope 50 and the inner surfaces of the respective grooves 24 and 26 provided on the inner circumferential surface of the body portion 22 of the cover 20. Therefore, the cross-sectional areas of the flow passages for the washing fluids can be increased, as compared to the endoscope unit according to the first embodiment shown in FIGS. 1 to 5 and the endoscope unit according to the second embodiment shown in FIGS. 19 to 23, and thus the amount of each washing fluid supplied to the observation window portion 54 of the rigid endoscope 50 per unit time can be increased.

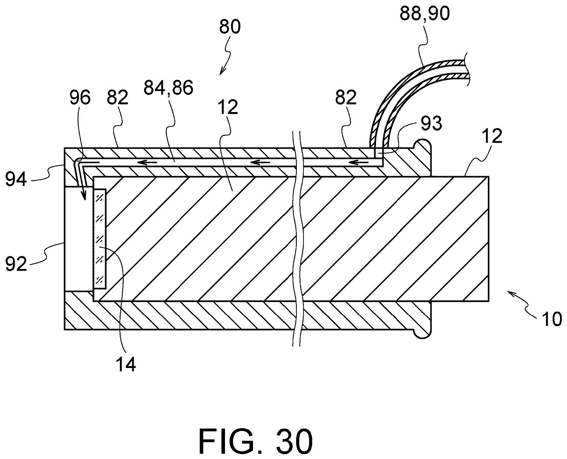

As still another example of the endoscope unit, an endoscope unit, in which, as flow passages for washing fluids, grooves are not provided on the inner circumferential surface of a cover covering the outer circumferential surface of a rigid endoscope but through holes are provided within a body portion of the cover, may be used. Such an endoscope unit will be described with reference to FIGS. 29 and 30. FIG. 29 is a cross-sectional view of an endoscope unit in which a cover 80 having through holes 84 and 86 provided within a body portion 82 is overlaid on the rigid endoscope 10 shown in FIG. 1, and FIG. 30 is a cross-sectional view of the endoscope unit shown in FIG. 29, taken along a virtual plane F or G passing through the center line of the rigid endoscope 10.

In the endoscope unit shown in FIGS. 29 and 30, the material of the hollow cover 80 for covering the outer circumferential surface of the rigid endoscope 10 is rubber such as natural rubber or synthetic rubber, and the cover 80 is overlaid on the outer circumferential surface of the rigid endoscope 10 shown in FIG. 1. More specifically, the cover 80 has the elongated substantially cylindrical body portion 82, and is overlaid on the outer circumferential surface of the rigid endoscope 10 by inserting the tip of the rigid endoscope 10 into a hollow portion at the base side of the body portion 82. In addition, the two through holes 84 and 86 are formed within the body portion 82 of the cover 80 so as to extend along the longitudinal direction of the body portion 82. Here, of the two through holes 84 and 86, the through hole 84 is used as a flow passage for washing water such as physiological saline to be supplied to the observation window portion 14 of the rigid endoscope 10, and the other through hole 86 is used as a flow passage for gas for blowing away the washing water adhering to the observation window portion 14. In the endoscope unit shown in FIGS. 29 and 30, the flow passages for the washing fluids are not formed at the gap between the rigid endoscope 10 and the cover 80, and the through holes 84 and 86 provided within the body portion 82 of the cover 80 themselves function as the flow passages for the washing fluids.

As shown in FIG. 30, an opening 92 is provided at a tip of the cover 80, and the observation window portion 14 is exposed to the outside of the cover 80 by the opening 92 when the cover 80 is overlaid on the outer circumferential surface of the rigid endoscope 10. In addition, a fluid direction change portion 94 that changes the direction of the washing fluids flowing within the respective through holes 84 and 86 (that is, along the longitudinal direction of the body portion 82 of the cover 80) to a direction toward the observation window portion 14 (that is, a direction toward the hollow portion of the cover 80), is provided near the opening 92 at the tip of the cover 80. Specifically, the fluid direction change portion 94 is provided with a curved portion 96 that defines the flow passages for the washing fluids, and the direction of the washing fluids is changed by the curved portion 96 from the left direction to a downward right direction in FIG. 30. Since the direction of the washing fluids flowing in the left direction in FIG. 30 along the outer circumferential surface of the body portion 12 of the rigid endoscope 10 can be changed by such a fluid direction change portion 94 to the direction toward the observation window portion 14 of the rigid endoscope 10, the observation window portion 14 can be reliably washed by the washing fluids. Moreover, such a fluid direction change portion 94 projects inward from the body portion 82 toward the hollow portion of the cover 80, and is formed such that the tip of the rigid endoscope 10 is caught on the fluid direction change portion 94 when the cover 80 is overlaid on the rigid endoscope 10. Therefore, the tip of the rigid endoscope 10 can be prevented from protruding from the tip of the cover 80.

As shown in FIG. 30, fluid supply pipes 88 and 90 are connected to the base end side of the body portion 82 of the cover 80, and through holes 93 are formed in the body portion 82 of the cover 80 at locations to which the respective fluid supply pipes 88 and 90 are attached. Therefore, when the cover 80 is overlaid on the rigid endoscope 10, the washing water and the gas can be supplied from these fluid supply pipes 88 and 90 through the through holes 93 to the respective through holes 84 and 86. In addition, a washing water supply source (not shown) is connected to the fluid supply pipe 88 via a washing water supply passage (not shown), and the washing water supplied from the washing water supply source to the washing water supply passage is sent to the fluid supply pipe 88. Therefore, the washing water can be supplied from the fluid supply pipe 88 to the through hole 84. Moreover, a gas supply source (not shown) is connected to the fluid supply pipe 90 via a gas supply passage (not shown), and the gas supplied from the gas supply source to the gas supply passage is sent to the fluid supply pipe 90. Therefore, the gas can be supplied from the fluid supply pipe 90 to the through hole 86.

In the endoscope unit shown in FIGS. 29 and 30 as well, the through holes 84 and 86 are provided within the body portion 82 of the cover 80, and the flow passages for the washing fluids such as the washing water and the gas to be supplied to the observation window portion 14 of the rigid endoscope 10 are defined by these through holes 84 and 86. The observation window portion 14 is allowed to be washed within the body of a patient, by providing the through holes 84 and 86 as the flow passages for the washing fluids within the body portion 82 of the cover 80 as described above. Since such a cover 80 is merely overlaid on the rigid endoscope 10, the cover 80 can be disposed of after every surgery. Thus, unlike the case where a washing device that supplies a washing fluid to the observation window portion 14 is attached to the rigid endoscope 10, time and effort to wash such a washing device itself before every surgery can be omitted. Moreover, since the material of the cover 80 is rubber, the cover 80 can have elasticity. Thus, the cover 80 can be brought into close contact with the outer circumferential surface of the rigid endoscope 10 when the cover 80 is overlaid on the rigid endoscope 10. Moreover, by bringing the cover 80 into close contact with the outer circumferential surface of the rigid endoscope 10, the cover 80 can be prevented from being detached from the rigid endoscope 10 while the endoscope unit is inserted into the body of a patient. Furthermore, in the case where the material of the cover 80 is rubber, action of overlaying the cover 80 on the rigid endoscope 10 and action of detaching the cover 80 from the rigid endoscope 10 can be easily performed. In addition, in the case where the material of the cover 80 is rubber, even when the endoscope unit comes into contact with a site or the like within the body of a patient, the site is inhibited as much as possible from being damaged by the endoscope unit.

In the cover 80 used in the endoscope unit shown in FIGS. 29 and 30, the through holes 84 and 86 are provided within the body portion 82. Thus, the thickness of the cover 80 is larger than the thickness of the cover 20, in which the grooves 24 and 26 are provided on the inner circumferential surface of the body portion 22 as shown in FIGS. 2 to 5, and the thickness of the cover 60, in which no groove is provided on the inner circumferential surface of the body portion 62 as shown in FIGS. 20 to 23. In addition, in the cover 80 used in the endoscope unit shown in FIGS. 29 and 30, the through holes 84 and 86 have to be formed within the body portion 82, and thus it is more difficult to produce the cover 80 than the cover 20, in which the grooves 24 and 26 are provided on the inner circumferential surface of the body portion 22, and the cover 60, in which no groove is provided on the inner circumferential surface of the body portion 62.

In each endoscope unit described above, two flow passages that are a flow passage for washing water such as physiological saline and a flow passage for gas for blowing away the washing water are illustrated as the flow passages for the washing fluids. However, the present invention is not limited to such an example. As the rigid endoscope cover or the endoscope unit according to the present invention, one having only one washing fluid flow passage may be used, or one having three or more washing fluid flow passages may be used.

## Claims

1. A rigid endoscope cover (120,220, 320, 420) for covering a part in a circumferential direction of an outer circumferential surface of a rigid endoscope (10) having an observation window portion (14) provided at a tip thereof, the cover (120, 220, 320, 420) comprising:
an outer cover portion (122, 222, 322, 422) configured to cover the part in the circumferential direction of the outer circumferential surface of the rigid endoscope (10) and having flexibility; and
an inner cover portion (123, 223, 323, 423) provided on an inner surface of the outer cover portion (122, 222, 322, 422) and configured to define a flow passage (124, 126, 224, 226, 324, 326, 424) for a washing fluid to be supplied to the observation window portion (14) of the rigid endoscope (10), wherein
the outer cover portion (122, 222, 322, 422) is configured to be mounted onto the outer circumferential surface of the rigid endoscope (10) by the outer cover portion (122, 222, 322, 422) bending, **characterized in that**
the cover (120, 220, 320, 420) has a C-shape when the cover (120, 220, 320, 420) is seen along the longitudinal direction thereof, to allow the rigid endoscope (10) to be fitted into the space of the C-shaped portion.

2. The rigid endoscope cover (120, 220, 320, 420) according to claim 1, wherein a material of the outer cover portion (122, 222, 322, 422) is a plastic material.

3. The rigid endoscope cover (220) according to claim 1 or 2, wherein the flow passage (224, 226) for the washing fluid to be supplied to the observation window portion (14) of the rigid endoscope (10) is defined between the outer circumferential surface of the rigid endoscope (10) and the inner cover portion (223).

4. The rigid endoscope cover (320) according to claim 1 or 2, wherein the flow passage (324, 326) for the washing fluid to be supplied to the observation window portion (14) of the rigid endoscope (10) is defined between the inner surface of the outer cover portion (322) and the inner cover portion (323).

5. The rigid endoscope cover (320) according to any one of claims 1 to 4, wherein a recess (322a) is formed on the inner surface of the outer cover portion (322), and the inner cover portion (323) is configured to be detachably mounted to the recess (322a) of the outer cover portion (322).

6. The rigid endoscope cover (320) according to claim 5, wherein the inner cover portion (323) is formed from an elastically deformable material, and has a shape that allows the inner cover portion (323) to be mounted to the recess (322a) of the outer cover portion (322) by the inner cover portion (323) deforming elastically.

7. The rigid endoscope cover (120, 220, 320, 420) according to any one of claims 1 to 6, wherein a material of the inner cover portion (123, 223, 323, 423) is rubber.

8. The rigid endoscope cover (120, 220, 320, 420) according to any one of claims 1 to 7, wherein a fluid direction change portion (134, 234, 334, 434) configured to change a direction of the washing fluid flowing along the flow passage (124, 126, 224, 226, 324, 326, 424) to a direction toward a hollow portion of the cover (120, 220, 320, 420) is provided.

9. An endoscope unit comprising:
a rigid endoscope (10) having an observation window portion (14) provided at a tip thereof; and
a rigid endoscope cover (120, 220, 320, 420) according to any of the preceding claims.

## Patentansprüche

1. Abdeckung (120, 220, 320, 420) für ein starres Endoskop, um einen Teil in einer Umfangsrichtung einer Außenumfangsfläche eines starren Endoskops (10), das einen Beobachtungsfensterabschnitt (14) hat, der an der Spitze vorgesehen ist, zu bedecken, wobei die Abdeckung (120, 220, 320, 420) Folgendes umfasst:
einen äußeren Abdeckabschnitt (122, 222, 322, 422), der konfiguriert ist, den Teil in der Umfangsrichtung der Außenumfangsfläche des starren Endoskops (10) abzudecken, und der eine Biegsamkeit aufweist; und
einen inneren Abdeckabschnitt (123, 223, 323, 423), der an einer Innenfläche des äußeren Abdeckabschnitts (122, 222, 322, 422) vorgesehen ist und konfiguriert ist, einen Strömungsdurchgang (124, 126, 224, 226, 324, 326, 424) für ein Spülfluid zu definieren, das dem Beobachtungsfensterabschnitt (14) des starren Endoskops (10) zugeführt werden soll, wobei
der äußere Abdeckabschnitt (122, 222, 322, 422) so konfiguriert ist, dass er an der Außenumfangsfläche des starren Endoskops (10) durch Biegen des äußeren Abdeckabschnitts (122, 222, 322, 422) angebracht wird,
**dadurch gekennzeichnet, dass**
die Abdeckung (120, 220, 320, 420) bei der Betrachtung der Abdeckung (120, 220, 320, 420) längs der Längsrichtung eine C-Form hat, damit das starre Endoskop (10) in den Zwischenraum des C-förmigen Abschnitts eingepasst werden kann.

2. Abdeckung (120, 220, 320, 420) für ein starres Endoskop nach Anspruch 1, wobei ein Material des äußeren Abdeckabschnitts (122, 222, 322, 422) ein Kunststoffmaterial ist.

3. Abdeckung (220) für ein starres Endoskop nach Anspruch 1 oder 2, wobei der Strömungsdurchgang (224, 226) für das Spülfluid, das dem Beobachtungsfensterabschnitt (14) des starren Endoskops (10) zugeführt werden soll, zwischen der Außenumfangsfläche des starren Endoskops (10) und dem inneren Abdeckabschnitt (223) definiert ist.

4. Abdeckung (320) für ein starres Endoskop nach Anspruch 1 oder 2, wobei der Strömungsdurchgang (324, 326) für das Spülfluid, das dem Beobachtungsfensterabschnitt (14) des starren Endoskops (10) zugeführt werden soll, zwischen der Innenfläche des äußeren Abdeckabschnitts (322) und dem inneren Abdeckabschnitt (323) definiert ist.

5. Abdeckung (320) für ein starres Endoskop nach einen der Ansprüche 1 bis 4, wobei eine Aussparung (322a) an der Innenfläche des äußeren Abdeckabschnitts (322) ausgebildet ist, und der innere Abdeckabschnitt (323) so konfiguriert ist, dass er an der Aussparung (322a) des äußeren Abdeckabschnitts (322) abnehmbar angebracht ist.

6. Abdeckung (320) für ein starres Endoskop nach Anspruch 5, wobei der innere Abdeckabschnitt (323) aus einem elastisch verformbaren Material gebildet ist und eine Form hat, so dass der innere Abdeckabschnitt (323) an der Aussparung (322a) des äußeren Abdeckabschnitts (322) angebracht werden kann, indem der innere Abdeckabschnitt (323) elastisch verformt wird.

7. Abdeckung (120, 220, 320, 420) für ein starres Endoskop nach einem der Ansprüche 1 bis 6, wobei ein Material des inneren Abdeckabschnitts (123, 223, 323, 423) Gummi ist.

8. Abdeckung (120, 220, 320, 420) für ein starres Endoskop nach einem der Ansprüche 1 bis 7, wobei ein Abschnitt (134, 234, 334, 434) zum Ändern einer Fluidrichtung vorgesehen ist, der konfiguriert ist, eine Richtung des Spülfluids, das längs des Strömungsdurchgangs (124, 126, 224, 226, 324, 326, 424) fließt, in eine Richtung zu einem hohlen Abschnitt der Abdeckung (120, 220, 320, 420) zu ändern.

9. Endoskopeinheit, die Folgendes umfasst:
ein starres Endoskop (10), das einen Beobachtungsfensterabschnitt (14) hat, der an der Spitze vorgesehen ist; und
eine Abdeckung (120, 220, 320, 420) für das starre Endoskop nach einem der vorhergehenden Ansprüche.

## Revendications

1. Gaine d'endoscope rigide (120, 220, 320, 420) destinée à recouvrir une portion dans une direction circonférentielle d'une surface circonférentielle extérieure d'un endoscope rigide (10) ayant une partie de fenêtre d'observation (14) agencée à une extrémité de celui-ci, la gaine (120, 220, 320, 420) comportant :
une partie de gaine extérieure (122, 222, 322, 422) configurée pour recouvrir la portion dans la direction circonférentielle de la surface circonférentielle extérieure de l'endoscope rigide (10) et ayant de la souplesse ; et
une partie de gaine intérieure (123, 223, 323, 423) agencée sur une surface intérieure de la partie de gaine extérieure (122, 222, 322, 422) et configurée pour définir un passage d'écoulement (124, 126, 224, 226, 324, 326, 424) pour un fluide de lavage à acheminer jusqu'à à la partie de fenêtre d'observation (14) de l'endoscope rigide (10), dans laquelle
la partie de gaine extérieure (122, 222, 322, 422) est configurée pour être montée sur la surface circonférentielle extérieure de l'endoscope rigide (10) par une flexion de la partie de gaine extérieure (122, 222, 322, 422),
**caractérisée en ce que**
la gaine (120, 220, 320, 420) a une forme en C lorsque la gaine (120, 220, 320, 420) est vue le long de la direction longitudinale de celle-ci, pour permettre d'insérer l'endoscope rigide (10) dans l'espace de la partie en forme en C.

2. Gaine d'endoscope rigide (120, 220, 320, 420) selon la revendication 1, dans laquelle un matériau de la partie de gaine extérieure (122, 222, 322, 422) est une matière plastique.

3. Gaine d'endoscope rigide (220) selon la revendication 1 ou 2, dans laquelle le passage d'écoulement (224, 226) pour le fluide de lavage à acheminer jusqu'à la partie de fenêtre d'observation (14) de l'endoscope rigide (10) est défini entre la surface circonférentielle extérieure de l'endoscope rigide (10) et la partie de gaine intérieure (223).

4. Gaine d'endoscope rigide (320) selon la revendication 1 ou 2, dans laquelle le passage d'écoulement (324, 326) pour le fluide de lavage à acheminer jusqu'à la partie de fenêtre d'observation (14) de l'endoscope rigide (10) est défini entre la surface intérieure de la partie de gaine extérieure (322) et la partie de gaine intérieure (323).

5. Gaine d'endoscope rigide (320) selon l'une quelconque des revendications 1 à 4, dans laquelle un évidement (322a) est formé sur la surface intérieure de la partie de gaine extérieure (322), et la partie de gaine intérieure (323) est configurée pour être montée de manière détachable sur l'évidement (322a) de la partie de gaine extérieure (322).

6. Gaine d'endoscope rigide (320) selon la revendication 5, dans laquelle la partie de gaine intérieure (323) est formée à partir d'un matériau élastiquement déformable, et a une forme qui permet de monter la partie de gaine intérieure (323) sur l'évidement (322a) de la partie de gaine extérieure (322) par une déformation élastique de la partie de gaine intérieure (323).

7. Gaine d'endoscope rigide (120, 220, 320, 420) selon l'une quelconque des revendications 1 à 6, dans laquelle un matériau de la partie de gaine intérieure (123, 223, 323, 423) est un caoutchouc.

8. Gaine d'endoscope rigide (120, 220, 320, 420) selon l'une quelconque des revendications 1 à 7, dans laquelle une partie de changement de direction de fluide (134, 234, 334, 434) configurée pour faire passer une direction du fluide de lavage s'écoulant le long du passage d'écoulement (124, 126, 224, 226, 324, 326, 424) à une direction vers une partie creuse de la gaine (120, 220, 320, 420) est prévue.

9. Unité d'endoscope comportant :
un endoscope rigide (10) ayant une partie de fenêtre d'observation (14) agencée à une extrémité de celui-ci ; et
une gaine d'endoscope rigide (120, 220, 320, 420) selon l'une quelconque des revendications précédentes.
